(19) Europäisches Patentamt
European Patent Office
Office européen des brevets

(11) EP 1 698 694 A1

(12) **EUROPEAN PATENT APPLICATION**
published in accordance with Art. 158(3) EPC

(43) Date of publication:
**06.09.2006 Bulletin 2006/36**

(21) Application number: **04736143.1**

(22) Date of filing: **04.06.2004**

(51) Int Cl.:
*C12N 15/09* (2006.01) *C12N 15/12* (2006.01)
*C12Q 1/68* (2006.01) *C07K 14/47* (2006.01)
*C07K 16/18* (2006.01) *C12P 21/02* (2006.01)
*C12N 1/15* (2006.01) *C12N 1/19* (2006.01)
*C12N 1/21* (2006.01) *C12N 5/10* (2006.01)

(86) International application number:
**PCT/JP2004/008174**

(87) International publication number:
**WO 2005/054465 (16.06.2005 Gazette 2005/24)**

(84) Designated Contracting States:
**AT BE BG CH CY CZ DE DK EE ES FI FR GB GR HU IE IT LI LU MC NL PL PT RO SE SI SK TR**

(30) Priority: **01.12.2003 JP 2003402306**
**14.01.2004 JP 2004006630**

(71) Applicant: **Post Genome Institute Co., Ltd**
**Tokyo 113-0033 (JP)**

(72) Inventors:
- **HASHIMOTO, Shin-ichi**
  **Tokyo 110-0001 (JP)**
- **MATSUSHIMA, Kouji**
  **Matsudo-shi,**
  **Chiba 271-0092 (JP)**
- **SUGANO, Sumio**
  **Tokyo 167-0052 (JP)**

(74) Representative: **Denison, Christopher Marcus**
**Mewburn Ellis LLP**
**York House**
**23 Kingsway**
**London WC2B 6HP (GB)**

(54) **METHOD OF OBTAINING GENE TAG**

(57) The present invention provides methods for providing as tags the nucleotide sequences at the 5' end of mRNA. The method of the present invention comprises the step of synthesizing cDNA using, as a template, mRNA whose CAP structure is linked with a IIs linker having a type IIs endonuclease recognition sequence. Tags including the nucleotide sequence from the 5' end of mRNA are generated by reacting the type IIs endonuclease to cDNA. Tags can be generated from all mRNA, independently of their nucleotide sequences. Methods for identifying transcriptional start sites and primers for full-length cDNA synthesis are provided based on the nucleotide sequence information of tags of the present invention.

EP 1 698 694 A1

## Description

### Technical Field

The present invention relates to methods for obtaining gene tags and methods for analyzing gene tags.

### Background Art

Cells can be characterized by comparing gene expression patterns among various cells. Specifically, cell catalogs can be prepared, in which cellular states are represented by gene expression patterns. With such catalogs, cells can be specified based on their gene expression patterns. Conversely, genes characteristic of each cell can be identified by comparing gene expression patterns between cells. For example, genes whose expression levels are altered upon an artificial treatment can be identified by comparing gene expression patterns between normal cells and cells subjected to the artificial treatment. Expression levels of such genes are altered as a result of the artificial treatment. Likewise, genes associated with a disease can be identified by comparing gene expression patterns between patient's cells and cells of healthy donors.

Comparing types and expression levels of genes between cells through exhaustive analysis of genes expressed in cells in a particular state by comparing gene expression patterns as described above is called "gene expression analysis". There exist various technologies for gene expression analysis.

For example, the methods following have been developed to isolate genes whose expression levels are different between cDNA libraries:

- Differential display and
- Subtraction library.

These methods have been used in practice for a relatively long time. Both are analytical methods for identifying genes whose expression levels differ among cDNA libraries of different origins. In recent years, nucleotide sequence information from a vast number of genes has been accumulated, and more efficient gene expression analyses have been achieved using the nucleotide sequence information. Specifically, DNA array technology has allowed for improved efficiency. In DNA array, tens of thousands of gene probes are arranged at high density. Expression patterns of tens of thousands of genes can be obtained simultaneously in a single experiment using a single DNA array. It is estimated that the total number of human genes is 30,000 to 40,000. Thus, DNA array has been used widely as a powerful tool to analyze human gene expression. In addition, DNA array has proven to be useful in the discovery of therapeutic targets and the development of candidate compounds for pharmaceuticals (Nature Genetics, volume 32, supplement pp. 547-552, 2002).

However, in general, probes constituting DNA array have been designed based on known nucleotide sequence information. Therefore, this device is inadequate for isolating unidentified genes. Furthermore, DNA arrays currently available in the market are limited to those derived from organisms for which gene sequence information has been sufficiently accumulated. For example, Affymetrics provides DNA arrays for the following species:

- Arabidopsis thaliana *(Arabidopsis* ATH1 Genome Array),
- Nematode (*C. elegans* Genome Array),
- Fruit fly (*Drosophila* Genome Array),
- E. coli (*E. coli* Antisense Genome Array),
- Human (Human Genome Focus Array and such),
- Mouse (Mouse Expression Set 430 and such),
- Pseudomonas (*P. aeruginosa* Genome Array),
- Rat (Rat Expression Set 230 and such), and
- Yeast (Yeast Genome S98 Array).

To perform DNA array-based gene expression analyses for other species, users must themselves prepare the DNA arrays, using a spotter or such. Otherwise, it is necessary for users to have the array custom prepared. However, it is difficult to prepare DNA arrays based on gene sequence information of species for which there is insufficient gene sequence data.

SAGE (serial analysis of gene expression) has been proposed as a technique that enables the isolation of unidentified genes and highly efficient gene expression analysis (SCIENCE, Vol. 270, 484-487, Oct. 20, 1995). SAGE is a technique used to obtain gene-specific tags and to carry out exhaustive analysis of the nucleotide sequences of the tags. A "gene tag" is a gene fragment that can be used as a label for the gene. In general, the probability that different genes share a completely identical nucleotide sequence of about 10 to 20 consecutive nucleotides is low. For example, in theory, one can discriminate among 262,144 (or $4^9$) types of genes using fragments of only 9 nucleotides. Thus, fragments of such length are useful as gene tags.

In the human genome sequence, the frequency of occurrence of a tag sequence consisting of 18 to 21 nucleotides and the probability that the tag sequence is specific to the nucleotide sequence of a gene can be calculated as follows:

- 18: once per 268,435,456 nucleotides (89.43%),
- 19: once per 1,073,741,824 nucleotides (97.24%),
- 20: once per 4,294,967,296 nucleotides (99.3%), and
- 21: once per 17,179,869,184 nucleotides (99.83%).

Thus, in theory, a tag sequence of 18 nucleotides can be presumed to have about 90% or higher probability as a nucleotide sequence specific to a gene, and a tag sequence of 20 nucleotides can be presumed to have about 99% or higher probability as a nucleotide sequence specific to a gene. A nucleotide sequence specific to a particular gene is referred to as "a unique nucleotide sequence of the gene". A nucleotide sequence whose frequency of occurrence in the genome is presumed to be 1 is referred to as "a unique nucleotide sequence in the genome".

In SAGE, gene tags are generated using the activity of a type IIs endonuclease. Such type IIs endonucleases that generate tags in SAGE are referred to as "tagging enzyme". Whereas a type II endonuclease cleaves DNA within its recognition sequence, a type IIs endonuclease cleaves DNA at a position apart from its recognition sequence. The distance between recognition sequence and cleavage position is almost constant for each enzyme. For example, *Bsm*FI or *Fok*I cleaves DNA at the position 9 to 10 nucleotides apart from its recognition sequence and generates sticky ends. Other known type IIs endonucleases having similar activity include the following enzymes (Szybalski, Gene 40:169,1985): *Bbv*I, *Bbv*II, *Bin*I, *Fok*I, *Hga*I, *Hph*I, *Mbo*II, *Mnl*I, *Sfa*NI*, Taq*II, and *Tth*lllII.

*Mme*I, a type IIs endonuclease, cleaves DNA at a position 20 nucleotides apart from its recognition sequence (5'-TCCRAC-3') (Tucholski et al, Gene, Vol.157, pp.87-92, 1995). A method of expression analysis, by which tags of 20 nucleotides can be obtained using *Mme*I as a tagging enzyme, is described in U.S. Patent No. 6,498,013. In particular, SAGE using *Mme*I is also referred to as "long SAGE". The principle of typical SAGE is summarized below.

First, cDNA is digested with a type II endonuclease, and the resulting fragment is recovered. When the recognition sequence for the type II endonuclease consists of 4 nucleotides, the cDNA is, in theory, cleaved into fragments every 256 nucleotides ($4^4$). For example, the *NlaIII* recognition sequence consists of 4 nucleotides. When cDNA is immobilized at its 5' or 3' end onto a solid phase, the resulting 5' or 3' fragment from the cleaved cDNA can be recovered easily. The recovered cDNAs are then divided into two reaction systems, and the treatment described below is carried out for each reaction system.

An adapter is ligated to the end of recovered cDNA resulting from the cleavage. Nucleotide sequences arranged in the adapter are as follows: a nucleotide sequence for a primer for PCR amplification at one end; an anchoring enzyme recognition sequence in the middle; and a type IIs endonuclease (tagging enzyme) recognition sequence at the other end, which is to be linked to cDNA. The cDNAs divided into two pools are separately linked with an adapter having a distinct nucleotide sequence for a primer. When a type IIs endonuclease is reacted after adapter ligation, the type IIs endonuclease recognizes the cDNA end and cleaves the cDNA at a position apart. Thus, a tag is generated, composed of a fragment from the position cleaved by the type II endonuclease up to the position cleaved by the type IIs endonuclease. The resulting tag has the adapter ligated.

The tag's sticky end generated through the cleavage with the type IIs endonuclease is converted to a blunt end by T4 DNA polymerase. Then, tags from the divided two reaction systems described above are ligated together at each blunt end. As a result, the two tags are linked together, facing each other and having different primer sequences arranged at their ends. The ligate of the two tags is referred to as "ditag". The ditag is amplified by PCR and cleaved with an anchoring enzyme. As a result, the primer sequences at the ends are removed from the PCR products. Next, the ditags without the primer sequences are linked together to generate ditag concatemers. The concatemers thus prepared are then inserted into sequencing vectors.

A number of nucleotide sequences of gene tags derived from multiple genes can be identified simultaneously by analyzing the nucleotide sequence of the concatemer. In theory, by bringing together nucleotide sequence information from concatemers obtained from a cDNA library, all gene tag information of cDNAs constituting the library can be obtained. Expression analyses can be readily achieved by comparing the tag information obtained as described above between cells.

Accumulated nucleotide sequence information is essential for DNA array-based expression analysis. Thus, commercially available DNA arrays are limited to those from some species, such as human, mouse, and yeast. Accordingly, to achieve DNA array-based gene expression analysis for many other species, one has to prepare new DNA arrays. DNA arrays use probes synthesized based on known nucleotide sequence information or cloned cDNAs as probes. Therefore, in general, it is difficult to find unidentified genes. In contrast, insufficient accumulation of nucleotide sequence information from genes is not an obstacle in carrying out SAGE analysis. In addition, SAGE requires no probe, and therefore is useful in isolating unidentified genes.

However, according to the protocols for SAGE as presently practiced, cDNA is digested with a restriction enzyme, and a linker composed of a type IIs endonuclease recognition sequence is linked at the cleavage position. Thus, the restriction enzyme recognition sequence used in SAGE must be short. If the recognition sequence of a restriction enzyme is long (i.e., a rare cutter), most cDNAs will not be cleaved by the enzyme. Thus, according to known SAGE, no tag is generated for cDNA that is not cleavable with a restriction enzyme.

For example, a restriction enzyme, such as *Nla*III, that recognizes 4 nucleotides, is expected to be suitable for SAGE. In theory, a cDNA has at least one unit of *Nla*III recognition sequence when its length is $4^4$ (=256) nucleotides or more. Although the probability that there exist transcripts of 256 nucleotides or less may be low, all cDNAs constituting a library do not necessarily include the *Nla*III recognition sequence. Specifically, even when the length of cDNA is 256 nucleotides or more, there is a possibility that a tag will not be generated. In fact, a report evaluating SAGE using nematode genes as a model has shown the existence of genes for which no tag is generated due to the lack of an *Nla*III recognition sequence (Genome Res. 2003 Jun. 13/6A:1203-15).

In addition, tags obtainable *via* this process are nucleotide sequences adjacent to the restriction site in the nucleotide sequence constituting cDNA. It is impossible to predict where the restriction enzyme recognition sequence exists in the cDNA of an unidentified gene. Specifically, it is unpredictable where the sequence information of a tag obtained by known SAGE is derived within the cDNA.

U.S. Patent No. 6,498,013 discloses that 5' and 3' tags are obtained when cDNA is immobilized at its 5' and 3' ends, respectively. However, tags generated *via* this process are composed of a nucleotide sequence adjacent to an endonuclease (*Nla*III) site located in the 5' or 3' region within cDNA. In other words, the sequence is the nucleotide sequence adjacent to the 5'- or 3'-furthermost endonuclease (*Nla*III) site among restriction sites contained in cDNA. Specifically, it is unclear where the resulting nucleotide sequence falls within the nucleotide sequence of cDNA.

In gene expression analyses, while it is not so important from which region of cDNA the nucleotide sequence constituting the tag is derived, if it can be determined which portion of cDNA the nucleotide sequence of the tag constitutes, the utility of the tag would be markedly increased. [Non-patent document 1] Nature Genetics volume 32 supplement pp 547-552, 2002

[Non-patent document 2] SCIENCE, Vol.270, 484-487, Oct. 20, 1995
[Non-patent document 3] Szybalski, Gene 40:169, 1985
[Non-patent document 4] Tucholski et al, Gene Vol.157, pp.87-92, 1995
[Non-patent document 5] Genome Res. 2003 Jun.13/6A:1203-15
[Patent document 1] US Patent 6498013

## Disclosure of the Invention

An objective of the present invention is to provide methods for obtaining gene tags and methods for analyzing gene tags, which are based on a novel principle.

As described above, in SAGE as presently practiced, a nucleotide sequence adjacent to a restriction enzyme recognition sequence is generated as a tag. Because of this, the relationship between the nucleotide sequence of the tag and the full-length cDNA sequence is difficult to understand. In addition, the problem of generating no tag for cDNA without such a restriction enzyme recognition sequence remains to be solved.

The present inventors conceived that the objectives described above would be achieved if tags could be produced independently of the presence of such a restriction enzyme recognition sequence. For example, if a tag is produced by using the 5' end ofmRNA, the nucleotide sequence of the tag can be expected to have various utilities. Thus, the inventors focused on the CAP structure that had been used in a method for synthesizing cDNA and examined its applicability in isolating gene tags. As a result, they discovered that the nucleotide sequence information from the 5' end of mRNA could be obtained as a tag, and thus completed the present invention. Specifically, the present invention relates to the following methods for obtaining tags and uses of such tags isolated by the methods.

[1] A method for producing a gene tag for eukaryotic cells, which comprises the steps of:

(1) linking an RNA linker, comprising a type IIs endonuclease recognition sequence, to the CAP site of an RNA;
(2) synthesizing a cDNA using the resulting RNA of (1) as a template; and
(3) reacting the resulting cDNA of (2) with a type IIs endonuclease that recognizes the recognition sequence in the RNA linker, and thereby generating the gene tag.

[2] The method of [1], wherein the cDNA is synthesized by the steps of:

(i) synthesizing a first strand of the cDNA using a primer that anneals to an arbitrary region of the RNA; and
(ii) preparing a double-stranded cDNA by synthesizing a second strand of the cDNA using a primer that anneals to a region of the first strand which has been synthesized using the RNA linker as a template.

[3] The method of [2], wherein the primer that anneals to a region of the first strand which has been synthesized using the RNA linker as a template has a label that binds to a solid phase or is immobilized onto a solid phase, wherein the method comprises the step of recovering the double-stranded cDNA by recovering the solid phase.
[4] The method of [3], wherein the solid phase is recovered before or after reaction with the type IIs endonuclease.
[5] The method of [1], wherein the RNA linker comprises a type II endonuclease recognition sequence.
[6] The method of [1], wherein the method comprises the step of generating a ditag by linking the end of a gene tag cleaved by the type IIs endonuclease to the end of another gene tag cleaved by the type IIs endonuclease.
[7] The method of [6], wherein the method comprises the step of amplifying the ditag using a primer that anneals to the RNA linker.
[8] The method of [1], wherein the method further comprises the step of ligating an adapter having an arbitrary nucleotide sequence to the end of a gene tag cleaved by the type IIs endonuclease and amplifying the gene tag using primers that anneal to the RNA linker and the adapter.
[9] A method for producing a concatemer of gene tags, wherein the method comprises the step of linking multiple gene tags generated by the method of [1].
[10] A method for producing a concatemer of gene tags, wherein the method comprises the step of linking multiple ditags generated by the method of [6].
[11] A method for determining the nucleotide sequence of a gene tag, wherein the method comprises the step of determining the nucleotide sequence of the concatemer described in [9] or [10].
[12] A reagent kit for producing a gene tag, wherein the kit comprises:

(a) an RNA linker that comprises an oligonucleotide comprising a type IIs endonuclease recognition sequence;
(b) a reagent for linking the RNA linker with the CAP site of an RNA;
(c) a primer for cDNA second strand synthesis, which comprises an oligonucleotide that anneals to a cDNA synthesized using the RNA linker as a template; and
(d) a primer for cDNA first strand synthesis.

[13] The kit of [12], wherein the primer for cDNA first strand synthesis is selected from the group consisting of:

(i) a random primer;
(ii) an oligo dT primer; and
(iii) a primer comprising a nucleotide sequence complementary to a particular mRNA.

[14] A method for obtaining an expression profile of a gene in eukaryotic cells, wherein the method comprises the steps of:

(1) producing a gene tag by the method of [1];
(2) determining the nucleotide sequence of the gene tag of (1); and
(3) obtaining the expression profile by relating the determined nucleotide sequence to its frequency of occurrence.

[15] A database of gene expression profiles constructed by accumulating information of gene expression profiles obtained by the method of [14].
[16] A method for analyzing gene expression profiles, wherein the method comprises the step of obtaining gene expression profiles from different types of cells by the method of [14], comparing the gene expression profiles and selecting a gene tag whose frequency of occurrence differs among the cells.
[17] A method for determining the transcriptional start site of a gene, wherein the method comprises the steps of:

(1) producing a gene tag by the method of [1];
(2) determining the nucleotide sequence of the gene tag of (1); and
(3) mapping the determined nucleotide sequence onto a genomic nucleotide sequence and identifying a region where the nucleotide sequences match as the transcriptional start site of the gene.

[18] The method of [17], wherein the primer for cDNA first strand synthesis comprises a nucleotide sequence selected from the nucleotide sequence of a particular gene, wherein the method comprises determining the transcriptional start site of the gene.
[19] A primer set for cDNA synthesis, wherein the primer set comprises a 3' primer that anneals to an arbitrary portion of a cDNA and a 5' primer for synthesizing a cDNA comprising a nucleotide sequence, or the complementary sequence thereto, determined by the steps of:

(1) producing a gene tag by the method of [1]; and
(2) determining the nucleotide sequence of the gene tag of (1).

[20] The primer set of [19], wherein the 3' primer is selected from the group consisting of:

(i) an oligo dT primer;
(ii) sequence information on a cDNA fragment; and
(iii) a primer comprising the nucleotide sequence of a gene tag adjacent to the type II endonuclease recognition sequence in the cDNA or the complementary sequence thereto.

[21] A method for synthesizing a full-length cDNA, wherein the method comprises the steps of:

(a) carrying out complementary strand synthesis using an RNA or cDNA as a template and using a 3' primer comprising an oligo dT primer and a 5'primer for synthesizing a cDNA comprising a nucleotide sequence, or the complementary sequence thereto, determined by the steps of:

(1) producing a gene tag by the method of [1]; and
(2) determining the nucleotide sequence of the gene tag of (1); and (b) recovering a synthesized DNA as the full-length cDNA.

[22] A full-length cDNA obtained by the method of [21].
[23] A polypeptide comprising an amino acid sequence encoded by the full-length cDNA of [22].
[24] An antibody which recognizes the polypeptide of [23].
[25] A vector carrying and capable of expressing the coding region of the full-length cDNA of [22].
[26] A transformant comprising and capable of expressing the vector of [25].
[27] A method for producing the polypeptide of [23], wherein the method comprises the step of culturing the transformant of [26] and collecting an expressed product.
[28] A method for producing the polypeptide of [23], wherein the method comprises the steps of:

(i) contacting an element supporting *in vitro* translation with a DNA construct comprising the coding region of the full-length cDNA of [22] operatively linked to a promoter; and
(ii) collecting an expressed product.

[29] A method for synthesizing a cDNA comprising a nucleotide sequence from the 5' end of an mRNA, wherein the method comprises the steps of:

(a) carrying out complementary strand synthesis using an RNA or cDNA as a template and using a 3' primer comprising a nucleotide sequence complementary to an arbitrary region of an mRNA of interest and a 5' primer for synthesizing a cDNA comprising a nucleotide sequence, or the complementary strand thereto, determined by the steps of:

(1) producing a gene tag by the method of [1]; and
(2) determining the nucleotide sequence of the gene tag of (1); and

(b) recovering a synthesized DNA as the cDNA comprising a nucleotide sequence from the 5' end of an mRNA.

[30] A method for determining the 5' nucleotide sequence of an mRNA, wherein the method comprises the step of determining the nucleotide sequence of the cDNA recovered by the method of [29].

The present invention provides methods for obtaining as gene tags the nucleotide sequences of the 5' ends of mRNAs. The 5' end of mRNA is a structure shared by all mRNA of eukaryotic cells. Thus, in principle, tags can be obtained from any gene, regardless of the nucleotide sequence of its mRNA. In contrast, SAGE based on the known principle provides as a tag a region adjacent to a restriction enzyme recognition site. Therefore, if the nucleotide sequence constituting the mRNA includes no restriction enzyme recognition site, one cannot obtain a tag for the gene. Thus, the present invention is significantly meaningful in that it ensures that tags may be obtained for all genes.

According to the tag method of the present invention, gene tags can also be obtained from mRNA fragments. RNA in biological samples is always at risk for degradation from various causes. Thus, isolation of cDNA or various analysis results yielded using the isolated cDNA largely depends on the condition of mRNA storage. Likewise, in SAGE, gene tags may not be obtained, or may not be reproducible, when mRNA structure is not completely maintained.

However, according to the method of the present invention, the 5' end of mRNA is obtained as a tag, and thus tags can be successfully obtained even in the case of fragmented mRNA, so long as the 5' end structure is intact. Thus, the method is hardly affected by mRNA storage conditions. This feature increases the reliability of gene expression analysis.

Furthermore, the nucleotide sequence of a tag obtained according to the present invention includes the nucleotide sequence from the 5' end of mRNA. Thus, the nucleotide sequence information of the tag which can be obtained according to the present invention is applicable in various fields. For example, the tag of the present invention may newly realize the following uses:

- Identification of transcription initiation sites in the genome,
- Providing primers for full-length cDNA synthesis, and
- Evaluation of cDNA library for the completeness.

It is unclear which region of mRNA corresponds to the nucleotide sequence of a tag isolated by SAGE based on the known principle. Therefore, such tags are not applicable to the uses described above.

The present invention relates to methods for producing gene tags for eukaryotic cells, which include the following steps of:

(1) linking an RNA linker including a type IIs endonuclease recognition sequence to the CAP site of RNA;
(2) synthesizing cDNA using the RNA of (1) as a template; and
(3) reacting the cDNA of (2) with a type IIs endonuclease that recognizes the recognition sequence in the RNA linker, and producing a gene tag including the sequence from the 5' end of RNA.

The CAP structure is the structure present at the 5' end of mRNA derived from eukaryotic cells or viruses infecting eukaryotic cells. Specifically, the CAP structure constitutes a 7-methylguanosine linked with the 5'-terminus nucleotide ofmRNA *via* a 5' to 5'-triphosphate crosslinkage. The CAP structure protects mRNA from degradation by 5'-3' exonuclease activity. Using a decapping enzyme, the CAP structure may be removed from mRNA that has outlived its usefulness in cells. As a result, such CAP structure-lacking mRNA is degraded by a 5'-3' exonuclease (LaGradeur et al., EMBO J., 17:1487-1496, 1998). The CAP structure is understood to be added to the 5' end of RNA at an early stage of transcription by RNA polymerase II.

The method of the present invention includes the step of linking an RNA linker to the CAP structure of RNA. Any type of RNA derived from eukaryotic cells can be used in the present invention. More specifically, it is possible to use polyA(+) RNA and total RNA. Moreover, it is possible to use cells derived from any species, such as animals, plants, yeast, and Myxomycetes, whose mRNA has the CAP structure.

RNA derived from viruses infecting such eukaryotic cells also has the CAP structure. Thus, in the context of the present invention, RNAs resulting from the transcription of gene information derived from eukaryotic cells, gene information infecting eukaryotic cells, and gene information introduced into eukaryotic cells are also included in the RNA derived from eukaryotic cells. The gene information infecting eukaryotic cells includes, for example, gene information of intracellular parasites, such as virus, viroid, and mycoplasma. Such gene information may be naturally occurring or artificially constituted. The gene information introduced into eukaryotic cells refers to information of artificially introduced genes *via* vectors or such. For example, even a gene of prokaryotic cells which is known to originally contain no CAP structure can be modified to have the CAP structure, by introducing the gene in a transcribable form into eukaryotic cells. Accordingly, RNA thus transcribed is also included in the RNA derived from eukaryotic cells in the context of the present invention.

RNA is extracted from such cells and used in the method of the present invention. Methods for extracting RNA are known. RNA extraction kits are commercially available and conveniently used. For example, high purity RNA can be obtained easily using a commercially available kit, such as RNAeasy (QIAGEN). When cell lysis is required to extract RNA, cells may be lysed by known methods.

In the context of the present invention, the RNA linker to be linked to the CAP structure includes at least an oligonucleotide having a type IIs endonuclease recognition sequence. The oligonucleotide to be used as the RNA linker may be DNA or RNA. The preferred RNA linker is RNA. The nucleotide sequence constituting the RNA linker may be any nucleotide sequence that includes the type IIs endonuclease recognition sequence. However, the type IIs endonuclease recognition sequence is preferably arranged at the 3' end of the RNA linker.

Type IIs endonucleases cleave at a position that is a fixed number of nucleotides apart from the recognition sequence. An aim of the present invention is to obtain the 5' end of mRNA as a tag. Thus, it is preferable that the recognition sequence be placed as closely to the 5' end of mRNA as possible. The type IIs endonuclease recognition sequence, which constitutes the RNA linker, can be designed to match the type IIs endonucleases to be used in the analysis. For example, as described above, the *Mme*I recognition sequence is 5'-TCCRAC-3' (R=G or A). This nucleotide sequence is thus arranged preferably at the 3' end of the RNA linker. The type IIs endonuclease recognition sequence is arranged so that the type IIs endonuclease cleaves it on the 3' side.

Nucleotide sequences useful as the RNA linker of the present invention are shown below. These nucleotide sequences are composed of a recognition sequence for *Xho*I, a type II endonuclease, (cucgag; underlined) in addition to the type IIs endonuclease (*Mme*I) recognition sequence (TCCRAC; capital letters) arranged at the 3' end.

5'-oligo 1 (SEQ ID NO: 1):

5'-uuuggauuugcuggugcaguacaacuaggcuuaauacucgagUCCGAC-3'

5'-oligo 2 (SEQ ID NO: 2):

5'-uuucugcucgaauucaagcuucuaacgauguacgcucgagUCCGAC-3'

The introduced *Xho*I site can be used to link the tag and to insert it into a vector. Furthermore, the nucleotide sequence constituting the RNA linker can also be used as a region to which a primer for tag amplification anneals. To achieve primer annealing, the region for the annealing is preferably composed at least 15 nucleotides, typically 20 to 50 nucleotides, for example, 20 to 30 nucleotides. The nucleotide composition can be designed so that the melting temperature (Tm) for the primer is typically in the range of 60 to 80°C, for example, in the range of about 65 to 75°C. The nucleotide sequence to which the primer anneals can be any nucleotide sequence. Thus, for example, it is possible to use an arbitrary nucleotide sequence giving the above Tm.

The nucleotide sequence to which the primer anneals may be any nucleotide sequence. The region constituting the recognition sequences for various endonucleases may overlap with the region for primer annealing in the RNA linker. However, when two types of RNA linkers are annealed with different primers, annealing specificity can be improved by designing them so as not to overlap each other.

In the present invention, the RNA linker is linked to the RNA CAP structure. Any method to link an oligonucleotide to the CAP structure can be used. For example, the oligo-capping method is a preferred method for linking the RNA linker of the present invention. The oligo capping method is a method developed for synthesizing cDNA containing the nucleotide sequence from the 5' end ofmRNA (Maruyama, K and Sugano, S.: Gene 138: 171-174, 1994). In the oligo capping method, full-length cDNA can be obtained using poly(A) sequence at the 3' end of mRNA and the nucleotide sequence of the RNA linker linked to the CAP structure at the 5' end. Since mRNA with an incomplete 5' nucleotide sequence has no CAP structure, the RNA linker is not linked to it. Thus, the oligo capping method can specifically yield full-length cDNA.

The reaction principle of the oligo capping method is described briefly below. First, mRNA is treated with bacterial alkaline phosphatase (BAP) to hydrolyze the phosphate group at the 5' end of RNA without the CAP structure. In this treatment, RNA without the CAP structure loses the phosphate group at the 5' end. Specifically, the phosphate group protruding at the 5' end of fragmented RNA, mitochondrial RNA, and such is removed. Then, the RNA is treated with tobacco acid pyrophosphatase (TAP). TAP hydrolyzes the triphosphate linkage of the CAP structure. As a result, only RNA with the CAP structure specifically has the 5' end phosphate group.

The RNA linker is then linked to the BAP- and TAP-treated RNA. The RNA linker can be ligated, for example, using T4 RNA ligase. The ligation using T4 RNA ligase requires the 5' end phosphate group. Thus, the RNA linker is ligated specifically to RNA having the 5' end phosphate group introduced by TAP. The RNA linker can thus be linked specifically with the CAP structure. All steps of reactions treating RNA are preferably performed in RNase-free environments.

Some variations of the oligo capping method have been reported. For example, a CAP-binding protein column-based method for purifying RNA having the CAP structure is known (Edery, L. et al., Mol. Cell Biol. 15: 3363-3371, 1995). By using this method, RNA with the CAP structure can be immobilized onto solid phase. RNA having the CAP structure can be recovered by TAP treatment after removing RNA having no CAP structure through washing the solid phase. The recovered RNA has phosphate groups at the 5' end, and therefore the RNA linker can be linked thereto without any additional treatment. Accordingly, the method using CAP binding protein requires no BAP treatment.

Next, cDNA is synthesized using, as a template, RNA linked with the RNA linker. Any method can be used to synthesize the cDNA. A representative cDNA synthesis method is described below.

In general, cDNA synthesis consists of two steps: first strand synthesis and second strand synthesis. The first strand synthesis is a reverse transcription using RNA as a template. The second strand is synthesized in a complementary strand synthesis reaction using as a template the first strand DNA synthesized first. For each step, several reactions are known, which are characterized by primers that start the reaction.

In the present invention, the first strand cDNA can be synthesized using a primer that anneals to an arbitrary region of RNA. DNA synthesis methods using reverse transcriptase activity and RNA as a template are known. Specifically, methods for synthesizing the first strand through primer extension, which use reverse transcriptase (RT) derived from MMLV, a mutant thereof, or such, are known. Mutant reverse transcriptases include a commercially available mutant (Superscript II, Gibco BRL) that lacks the original RNaseH activity of reverse transcriptase. In addition, enzymes, such as Tth DNA polymerase, which are DNA synthesizing enzymes but also catalyze complementary strand synthesis from RNA template, are also known. When such an enzyme is used, both the first (RNA template) and second (DNA template) strands can be synthesized using the single enzyme. Primers for cDNA synthesis are described below.

In the oligo capping method described above, oligo dT primer is generally used in the first strand synthesis. To synthesize full-length cDNA, oligo dT primer having a nucleotide sequence complementary to poly(A) at the 3' end of mRNA is used because the first strand should be synthesized from its 3' end. Likewise, in the context of the present invention, the 5' end of full-length cDNA can be obtained as a tag sequence using the oligo dT primer.

However, the present invention does not require the use of full-length RNA. In the context of the present invention, tags are obtained from a short region including the 5' end of RNA. Thus, as long as cDNA can be synthesized from the region including the 5' end of RNA, it will be sufficient for use in the present invention. Thus, the first strand can be synthesized, for example, using a random primer that can start complementary strand synthesis from an arbitrary position in RNA. By using a random primer, a tag can be obtained even from a fragment whose nucleotide sequence is incomplete at the 3' end, so long as the RNA has the CAP structure. The random primer is particularly useful in gene expression analysis because it allows tags to be obtained from a broader range of RNA.

Furthermore, a tag for a particular gene can be selectively obtained using a primer that includes a nucleotide sequence complementary to the nucleotide sequence of the particular gene in the first strand synthesis. For example, according to the present invention, even when only a partial nucleotide sequence of a gene is known but the 5' nucleotide sequence remains unidentified, its 5' end tag sequence can be obtained. For this purpose, a nucleotide sequence of a primer for the first strand synthesis is selected from the known nucleotide sequence. With this primer, a region spanning from the identified region to the 5' end of mRNA is generated as the first strand of cDNA. Since the primer is selected from the nucleotide sequence of the particular gene, the first strand is not generated from RNA for genes other than the target gene. Thus, tags for such genes are not generated.

A gene tag for a particular target gene obtained by the method of the present invention can be expected to have, for example, the following utilities. First, the transcriptional start site of the gene can be identified from the nucleotide sequence information of the obtained gene tag. The transcriptional start site information is important to obtain full-length cDNA or to search for the promoter. For example, when the 5' nucleotide sequence of a cDNA remains unidentified, a cDNA on the 5' side can be obtained using the method of the present invention. When the translation start site of a gene has already been identified, gene tag information can be used to evaluate whether its 5' untranslated region (5' UTR) is complete.

Furthermore, some genes have been found to produce multiple transcripts with different transcriptional start sites which encode an identical amino acid sequence. Gene tags of the present invention for a particular target gene may be obtained from various mRNA sources, and conveniently used to collect information on transcriptional start sites of all transcripts of the gene. If multiple types of gene tags are obtained for a gene, the gene is likely to have multiple transcripts with different transcriptional start sites. Specifically, the present invention provides methods for detecting multiple transcripts with different transcriptional start sites, which include the following steps:

(1) obtaining gene tags according to the present invention using a primer for cDNA first strand synthesis that is specific to a gene to be analyzed;
(2) comparing the nucleotide sequences of the gene tags obtained in step (1); and
(3) detecting multiple transcripts with different transcriptional start sites when multiple types of gene tags are detected.

The nucleotide sequence at the transcriptional start site of each transcript can be determined using information of the above-described gene-specific primer and multiple types of gene tags detected according to the present invention. In addition, the expression levels of respective transcripts can be compared according to the present invention. Specifically, the present invention provides methods for comparing the expression levels of multiple transcripts with different transcriptional start sites, which include the following steps:

(1) obtaining gene tags according to the present invention and using a primer for cDNA first strand synthesis that is specific to a gene to be analyzed;
(2) comparing the nucleotide sequences of the gene tags obtained in step (1); and
(3) determining expression levels of multiple transcripts with different transcriptional start sites based on the frequency of occurrence of each gene tag.

Furthermore, cDNAs can be intentionally synthesized from RNAs of the same nucleotide sequence. For example, a primer for first strand synthesis can be designed based on a nucleotide sequence that is predicted to encode an amino acid sequence that constitutes a highly conserved functional domain of a protein. cDNA synthesized using this primer is likely to be cDNA of a gene encoding the particular functional domain. Thus, tags of genes having the particular functional domain can be deliberately selected. The expression levels of a group of genes having a particular function can be compared by comparing the expression levels of gene tags obtained as described above.

In any case, the first strand of cDNA synthesized according to the present invention includes at its 3' end a nucleotide sequence complementary to the RNA linker. Thus, the second strand of cDNA can readily be synthesized using an oligonucleotide capable of annealing to this region. Prior to the second strand synthesis, RNA used as a template for the first strand can be removed through alkaline hydrolysis. In the context of the present invention, the second strand should be synthesized so as to include at least the type IIs endonuclease recognition sequence included in the RNA linker. To achieve this goal, it is possible to use, for example, a primer that starts the complementary strand synthesis from a position more 3'-side than a region corresponding to the type IIs endonuclease recognition sequence arranged at the 3' end of the RNA linker. Alternatively, it is possible to use a primer including the type IIs endonuclease recognition sequence.

Methods for synthesizing complementary strands through primer extension using DNA as a template are known. Specifically, methods for synthesizing complementary strands using a template-dependent DNA polymerase are known. T4 DNA polymerase, Taq polymerase, and the like can be used as the DNA polymerase.

Primers to be used in cDNA synthesis may include an arbitrary nucleotide sequence. For example, it is possible to use a primer having an endonuclease recognition sequence at its 5' end. Addition of a nucleotide sequence containing cloning sites to the 5' end of a primer has widely been performed.

In the context of the present invention, the second strand of cDNA may have a label capable of binding to a solid phase or can be synthesized using a primer immobilized onto such a solid phase. The second strand of cDNA can be captured by the solid phase via primers immobilized thereto. cDNA captured by the solid phase can then be easily recovered.

Any method can be used to immobilize oligonucleotides to be used as the primers onto the solid phase. For example, a method for covalently linking the 5' end of an oligonucleotide to plates through use of a cross-linker is described in U.S. Patent No. 5,656,462. Alternatively, it is possible to introduce molecules having a binding affinity, such as biotin, into nucleotides constituting the oligonucleotide. The oligonucleotide is captured indirectly by solid phase through biotin with avidin immobilized onto the solid phase. The position of introduction of the molecule with binding affinity in the oligonucleotide is not particularly limited.

The double-stranded cDNA from the second strand synthesis is treated with a type IIs. endonuclease to generate the gene tags of the present invention. At this stage, the gene tags can be recovered in a form linked with the nucleotide sequence attached as the RNA linker. The solid phase onto which primers for the second strand synthesis have been immobilized is then used to recover the gene tags. Specifically, the gene tags are recovered as gene tag-bound solid phase. The solid phase can be recovered before or after treatment with the type IIs endonuclease.

Nucleotide sequence information of the 5' end of RNA can be obtained by determining the nucleotide sequence of a gene tag of the present invention. Any method can be used to determine the nucleotide sequence of a gene tag. However, the principle of SAGE is useful for the efficient determination of the nucleotide sequences of a vast number of gene tags. Specifically, when multiple gene tags are linked together as a concatemer and such concatemers are cloned, the nucleotide sequences of multiple tags can be determined simultaneously.

Due to the action of the type IIs endonuclease used to produce the tags, the length of each gene tag is presumed to be constant. Thus, the concatemers can be considered to be constituted by repeats of the nucleotide sequences of gene tags having a fixed length. Nucleotide sequence information of each tag can thus be obtained from the nucleotide sequences of the concatemers.

There are some variations to the method for obtaining concatemers by ligating tags, examples of which are described below. First, a method using the well-known SAGE principle is described. In this method, two gene tags are first ligated together in opposite directions to yield a ditag. If the end resulting from cleavage by the type IIs endonuclease is a sticky end, it should be blunted prior to this step. The blunting can be achieved using T4 DNA polymerase.

Next, multiple ditags are linked together to yield concatemers. To prepare ditags, a cDNA library is divided into two pools, and then gene tags are produced from each pool by the same procedure. Then, gene tags derived from the two pools are ligated together to yield ditags. At this stage, gene tags are ligated at the position where the type IIs endonuclease cleaved. The gene tags can be ligated together enzymatically, with T4 DNA ligase or the like.

Ditags yielded herein have the following structure.

PCR→

(solid phase)-[RNA linker]-[tag]-[tag]-[RNA linker]-(solid phase)

←PCR

At this stage, the ditags can be amplified by amplification methods, such as PCR. When the nucleotide sequence of the RNA linker is designed to be different between the two pools, only ditags resulting from ligation of tags from the different pools are specifically amplified, thereby preventing imbalance in number between tags. In the context of the present invention, the ditag may or may not be amplified.

Next, multiple ditags are ligated together to yield concatemers. To achieve this goal, for example, an endonuclease recognition sequence may be arranged in advance within the RNA linker. Multiple ditags can be linked together by ligating ditag ends after cleaving the ditags with an endonuclease. The structure of concatemers thus obtained is shown below. ...../[Tag] [Tag]/[Tag] [Tag]/[Tag] [Tag]/[Tag] [Tag]/....

Specifically, taking two tags linked together (ditag "[Tag] [Tag]") as one unit, the structure is composed of connected ditags with intervening endonuclease (anchoring enzyme) cleavage sites ("/").

The concatemer can be inserted into a cloning vector at the same restriction site. A cloning vector carrying the concatemer as an insert can thus be prepared. The nucleotide sequence of the tags in the cloning vector is revealed by determining the nucleotide sequence of the insert in the vector. It is preferred that the length of the concatemer is within a range that allows the determination of the nucleotide sequence in a single sequencing reaction. For example, the concatemer is 500 bp or shorter, for example, in the range of 20 to 400 bp, typically in the range of 50 to 300 bp.

It is also possible to obtain concatemers composed of tags linked together in tag units instead of ditags. For example, after type IIs endonuclease treatment, an adapter can be attached to the end resulting from the cleavage. In this case, the tag has the following structure.

PCR→

(solid phase)-[RNA linker]-[tag]-[adapter]

←PCR

When an endonuclease recognition sequence is arranged in the adapter, both ends of the tag can be cleaved with the endonuclease in the same way that the RNA linker of ditag is digested. When amplifying the tag, PCR using the nucleotide sequences of the RNA linker and adapter may be used. In any case, tags treated with the endonuclease can be linked together to yield concatemers. The concatemers can then be inserted into a cloning vector to determine their nucleotide sequences.

The lengths of tags excised by a type IIs endonuclease are considered to be relatively constant. However, if tags of variable length result and ditags are formed from such tags, the nucleotide sequences of the tags may not be correctly identified. When the concatemer is constructed not *via* the ditag, the nucleotide sequence of tag can be determined accurately even if the lengths of tags are not uniform.

Various reagents necessary for the method for obtaining gene tags of the present invention and the method for determining the nucleotide sequences of the obtained tags can be combined together in advance and supplied as a kit. Specifically, the present invention relates to reagent kits for producing gene tags, which include the following elements:

(a) an RNA linker that includes an oligonucleotide having a recognition sequence for a type IIs endonuclease;
(b) a reagent for linking the RNA linker with the CAP site of RNA;
(c) a primer for cDNA second strand synthesis, which includes an oligonucleotide that anneals to cDNA synthesized using the RNA linker as a template; and
(d) a primer for cDNA first strand synthesis.

The kit of the present invention may additionally include reagents required to prepare ditags and/or concatemers. The specific constitution of these components is as described above.

For the kit of the present invention, for example, any of the following primers, described below in (i) to (iii), can be used as the primer for cDNA first strand synthesis of (d):

(i) a random primer;
(ii) an oligo dT primer; and

(iii) a primer having a nucleotide sequence complementary to a particular mRNA.

Random primers or oligo dT primers are used to generate gene tags from all mRNA in a sample. In the context of the present invention, the random primer is particularly preferred. The random primer is a set of oligonucleotides, each composed of a random nucleotide sequence whose length is several tens of nucleotides. For example, oligonucleotides of about 5 to 20 nucleotides, typically of about 8 to 15 nucleotides, may be used. These oligonucleotides are synthesized by sequentially linking the four types of nucleotides in a mixture until they reach a desired length. In theory, the random primer can include nucleotide sequences complementary to any kind of nucleotide sequence.
Alternatively, the kit of the present invention may be made up of primers having a nucleotide sequence complementary to a particular mRNA. 5' tags for a certain gene can specifically be produced using primers specific to a particular mRNA. If nucleotide sequence variations are detected in comparison of the nucleotide sequence information among tags obtained as described above, transcripts of the gene are found to include multiple variants whose lengths are different at the 5' end. Thus, the kit of the present invention, which is made up of primers having the nucleotide sequence complementary to a particular mRNA, is useful for detecting variant transcripts of a particular gene.
For example, the kit that is used to conduct the method of the present invention may include the elements listed below. A buffer suitable for a reaction using each element may be attached to each element. In addition, software for analyzing the nucleotide sequences of gene tags may be combined with the kit of the present invention.
Elements to ligate the RNA linker:

- BAP,
- TAP,
- T4 RNA ligase, and
- RNA linker.

Elements for cDNA synthesis and separation:

- reverse transcriptase,
- DNA polymerase,
- dXTP,
- random primer for cDNA first strand synthesis,
- 5'-biotinylated primer for cDNA second strand synthesis, and
- avidin-conjugated magnetic beads.

Elements to generate gene tags:

- type IIs endonuclease

Elements to generate and analyze ditags:

- T4 DNA ligase,
- primer for gene tag amplification,
- DNA polymerase,
- type II endonuclease,
- sequencing vector,
- host to be transformed with a vector, and
- culture medium for the host.

It is advantageous to use computer software in the analysis of the nucleotide sequence information on concatemers generated according to the present invention. The nucleotide sequence information of concatemers may be analyzed, for example, using software that can execute the steps of:

- reading data analyzed by a sequencer;
- distinguishing nucleotide sequence information other than that of tags in the input nucleotide sequence data; and
- accumulating nucleotide sequence information of tags,

Herein, the nucleotide sequence information other than that of tags includes, for example, nucleotide sequence information on the RNA linker and adapter linked in the process of generating the tags. The input data may also include nucleotide sequences derived from cloning vectors. In any case, such nucleotide sequence information constitutes previously known information. Such additional nucleotide sequence information and nucleotide sequence information of the tag are arranged in the concatemer in a regular fashion. Therefore, such nucleotide sequences can be automatically distinguished from the nucleotide sequences of the tag.

Next, nucleotide sequence information identified as the tag nucleotide sequences is accumulated. When the concatemer is made through ditags, some of the input nucleotide sequences may be derived from antisense strands. Therefore, information of the complementary sequence should be simultaneously recorded. When the concatemer is prepared using an adapter not *via* ditag, the tags can be cloned in one direction by designing the adaptor and the RNA linker to have different cloning sequences. In this case, it is unnecessary to accumulate complementary sequences.

This analysis program may have additional functions. For example, the program may facilitate comparison among the nucleotide sequences of obtained tags, accumulation of identical nucleotide sequences, and recordation of the frequency of their occurrence. Furthermore, the program may facilitate comparison of the tag information from different RNA sources and extraction of tags with different frequencies of occurrence.

Previously accumulated information from a database may be used in comparing tag information. For example, information of gene tags for major tissues and cell lines may be accumulated in advance according to the method of the present invention. Such information can be shared in a computer network. Alternatively, the information may be supplied commercially, being attached to the above-described reagent kit. Such available gene tag information may be compared with gene tag information obtained by experimenters themselves.

The nucleotide sequence information of the 5' end of transcript mRNA can be obtained according to the present invention. Such nucleotide sequence information of the 5' end is particularly important in the context of gene analysis. For example, nucleotide sequence information of the 5' end which can be obtained according to the present invention may have the utilities described below.

First, the present invention can be used in gene expression profiling. Specifically, the present invention relates to methods for obtaining expression profiles of genes in eukaryotic cells, which include the following steps of:

(1) producing gene tags according to the present invention;
(2) determining the nucleotide sequences of the gene tags of (1); and
(3) obtaining expression profiles by relating the determined nucleotide sequences to the frequency of their occurrence.

In the present invention, the step (1) of producing gene tags may include the steps described below. Likewise, hereinafter unless otherwise stated, "the step of producing gene tags according to the present invention" includes the following steps:

(A) linking an RNA linker having a type IIs endonuclease recognition sequence to the CAP site of RNA;
(B) synthesizing cDNA using the RNA of (A) as a template; and
(C) generating gene tags by reacting the cDNA of (B) with the type IIs endonuclease that recognizes the recognition sequence in the RNA linker.

In general, the term "expression profile" refers to a list of gene information including expressional information. The expressional information is a quantitative parameter representing the expression level. In general, the gene information refers to information to specify genes. Specifically, the gene information is made up of nucleotide sequences of genes, gene names, gene ID Nos., and the like. The number of genes included in the list is not particularly limited. The types of genes included in the list are also not limited. Depending on the purpose of analysis, desired genes' information may be accumulated to constitute expression profiles.

According to the present invention, the nucleotide sequence information of the 5' end of RNA can be obtained as tag information from RNA with the CAP structure. The nucleotide sequence information can be related to the frequency of occurrence by comparing the nucleotide sequence information and determining the number of identical nucleotide sequences. Expression profiles can thus be obtained.

An expression profile for an entire set of genes can be obtained when all RNAs are used as the targets. In the present invention, gene tags may also be generated for a specific gene or a group of genes sharing a common structure. In this case, an expression profile of the specific gene or the group of genes is produced.

Assuming that mRNA having the CAP structure includes all mRNA expressed in cells, an expression profile obtained according to the present invention more precisely reflects the pattern of gene expression in cells. In the context of the present invention, when determining the frequency of occurrence of a nucleotide sequence, the data is preferably accumulated as a value relative to the total number of nucleotide sequences being analyzed. In particular, after the sequences have been amplified by PCR or such, quantitative data is not meaningful. Comparison to the total number can be expected to provide more objective evaluation.

A database can be constructed from such expression profiles obtained according to the present invention.

Herein, a database refers to a set of electronic data including information constituting expression profiles accumulated as machine-readable data. The database of the present invention includes at least the nucleotide sequence information of the tags and information regarding the frequency of occurrence of each of the sequences. Furthermore, the ID No. of each nucleotide sequence and the origin of RNA whose nucleotide sequence information has been obtained may also be recorded in the database of the present invention. In addition, relation of the obtained nucleotide sequence information to that of known genes, results of mapping in the genome, and the like may be added to the database.

The expression profile database of the present invention can be stored as electronic media. Such electronic media include, for example, various disk devices, tape media, and flash memories. Such electronic media can be shared on a network. For example, a database of the present invention can be shared on the Internet. Furthermore, the above-described software for analyzing tag sequences can be provided with a function to refer to information in the database of the present invention *via* the Internet. Conversely, information of expression profiles newly generated according to the present invention may be added to the database *via* the Internet.

The analyses of expression profiles can be carried out using expression profiles of the present invention. Specifically, the present invention relates to methods for analyzing gene expression profiles, which include the steps of obtaining gene expression profiles for different types of cells and selecting gene tags whose frequency of occurrence is different between cells through comparing the expression profiles generated in accordance with the present invention. Such a method for analyzing genes whose expression levels are different between different cells is referred to as expression profile analysis. Many genes, for example, genes associated with diseases, have been identified through such analyses. The expression profile of the present invention can be used in such expression profile analyses.

In the expression profile analysis of the present invention, the "different cells" to be analyzed may be any cells of different origin. Even cells derived from the same tissue can be the cells of different origin, so long as they involve different conditions, such as the presence of disease, race, age, and sex. When conditions to be considered, depending on the purposes for the analysis, are different among cells, such cells are the cells of different origin. When differences in conditions are negligible for the purposes of the analysis, such cells are considered to be identical cells. For example, genes whose expression levels are high (or low) in organs, tissues, or cells can be selected by comparing expression profiles among different organs, different tissues, or cells whose origins, culture conditions, or such are different. Examples of combinations of analysis targets to which the present invention is applicable are shown below.

Different tissues:

Adult and fetal tissues,
Tissues of patient and healthy donor,
Tissues from male and female,
Human tissues derived from different races, and
Tissues of individuals belonging to the same species but grown in different environments.

Different cells:

Cells whose types are identical but culture conditions are different,
Cells cultured under the same culture conditions but whose culture periods are different, and
Cells subjected to a particular treatment and non-treated cells.

More specifically, gene tags characteristic of a cancer can be obtained by comparing the expression profile between cancer and normal tissues. Alternatively, gene tags associated with malignancy can be specified by comparing high- and low- malignancy cancers.

Gene tags obtainable according to the present invention include the nucleotide sequence information of the 5' end of mRNA. Therefore, variants of a gene, which encode an identical protein but are different in the structure of the 5' UTR, can be identified as different transcripts in the expression profile. This feature is one of the major advantages of the tags of the present invention as compared to tags obtained using conventional SAGE. In addition, the nucleotide sequence information of the gene tags of the present invention is useful by itself as the nucleotide sequence information of the primer at the 5' side of full-length cDNA. Thus, full-length cDNA can be easily synthesized using the oligo dT primer and primers designed based on the nucleotide sequence information of the tags obtained through expression profile analysis. Alternatively, by combining the 5'-side primer with a primer having a nucleotide sequence complementary to any one region of mRNA, cDNA composed of the nucleotide sequence from the 5' end of mRNA can be obtained. This is a further significant advantage of the present invention.

Gene tags that can be obtained according to the present invention include nucleotide sequences from the 5' end of transcript mRNA. Thus, the transcriptional start site of a gene can be identified by mapping the nucleotide sequence onto the genome nucleotide sequence. Specifically, the present invention relates to methods for determining the transcriptional start site of a gene, which include the following steps of:

(1) producing gene tags according to the method of the present invention;
(2) determining the nucleotide sequences of the gene tags of (1); and
(3) mapping the determined nucleotide sequences onto the nucleotide sequence of a genome and identifying the transcriptional start site of the gene as the region where the nucleotide sequences match.

In April 2003, The International Human Genome Sequencing Consortium announced the completion of the Human Genome Project. Thus, an accurate human genome sequence covering 99% of the entire human genome (2.83 billion base pairs) with 99.99% accuracy is presently available. Meanwhile, the present invention produces a tag from the 5' end of every mRNA transcribed in cells. Thus, in principle, almost all transcriptional start sites of genes transcribed in particular cells can be mapped onto the genome. Mapping transcriptional start sites to the genome is useful in identifying transcriptional regulatory regions.

For example, a region of 1 to 2 kb upstream of a transcriptional start site can be cloned and then used to screen for transcriptional regulatory factors. The nucleotide sequence of this region may also be analyzed to predict the transcriptional regulatory region. More specifically, regions where transcription factors bind can be predicted by searching for conserved regions among recognition sequences of known transcription factors.

Mapping of a transcriptional start site is equivalent to mapping of a gene. Specifically, the physical positional relationship of genes in the genome can be understood based on a result obtained by mapping the nucleotide sequence information of a tag in accordance with the present invention. Previously, the transcriptional start site of a gene could not be mapped without nucleotide sequence information of a high-quality full-length cDNA. However, transcriptional start sites can readily be mapped using tag information obtained in accordance with the present invention. Thus, it can be said that information of a tag obtained in accordance with the present invention is as valuable as that from the result obtained using full-length cDNA.

In addition, nucleotide sequence information of gene tags obtained in accordance with the present invention can be used to evaluate cDNA completeness. While genomic nucleotide sequences have been revealed, various attempts are now being made to clarify cellular functions at the protein level. One of such attempts is involves exhaustive full-length cDNA analysis. In this exhaustive full-length cDNA analysis, genes expressed in particular cells are exhaustively obtained as whole complete genes, and their structures are determined. To achieve this goal, it is important to have a high degree of completeness among the obtained cDNA.

First, at least the nucleotide sequence of 5'-side of mRNA is required to specify the ORF. Furthermore, it is important that the sequence is obtained up to its 5' end to identify the transcriptional start site. The completeness of obtained cDNA is often evaluated to confirm that the requirements described above are fulfilled. The cDNA completeness is a parameter that represents the proportion of cDNAs including the nucleotide sequence from the 5' end of mRNA to the total number of obtained cDNAs.

Gene tags of the present invention provide the nucleotide sequence information of the 5' end of mRNA. Thus, by comparing the nucleotide sequences of an exhaustive collection of cDNA with the nucleotide sequences of the gene tags of the present invention obtained from the same library, it can be determined whether the 5' end of each cDNA includes the nucleotide sequence from the 5' end of mRNA. When most of the nucleotide sequences of gene tags can be mapped onto the nucleotide sequences of cDNAs, most of the obtained cDNAs are likely to be full-length. Conversely, when nucleotide sequences corresponding to gene tags cannot be found in the obtained cDNAs, the completeness of the cDNAs is predicted to be low.

The nucleotide sequence information of gene tag of the present invention can be used to obtain cDNA composed of the nucleotide sequence from the 5' end of mRNA. Specifically, the present invention relates to primer sets for cDNA synthesis, which include a 3' primer that anneals to an arbitrary portion of cDNA and a 5' primer for synthesis of cDNA having a nucleotide sequence, or the complementary sequence thereto, determined by the steps of:

(1) producing gene tags according to the present invention; and
(2) determining the nucleotide sequences of gene tags of (1).

The nucleotide sequence of the 5' primer which constitutes the primer set of the present invention, includes a nucleotide sequence obtained as a tag or the complementary sequence thereto. Since a tag may be obtained as a sense sequence or an antisense sequence to mRNA, the nucleotide sequence of a tag itself or its complementary sequence may be used as the nucleotide sequence of 5' primer for cDNA synthesis'. Since the 5' primer initiates the complementary strand synthesis at the 5' end, cDNA synthesized using the primer set of the present invention includes the nucleotide sequence of the 5' end with no exception. The tag sequence includes "t" nucleotides because it is derived from DNA. It is needless to say that the nucleotide that corresponds to the nucleotide "t" is "u" in the 5' end sequence of RNA'.

Any primer capable of annealing to cDNA can be used as the primer on the 3' side that constitutes the primer set of the present invention. Various cDNAs can be synthesized depending on the type of selected 3' primer. The 3' primer that can be used for the primer set of the present invention includes, for example, the following primers:

(i) an oligo dT primer;
(ii) sequence information of a cDNA fragment; and
(iii) a primer having the nucleotide sequence of a gene tag adjacent to the type II endonuclease recognition sequence or the complementary sequence thereto in the cDNA.

First, combinations with the oligo dT primer are useful to synthesize full-length cDNA. Second, the primer on the 3' side designed based on sequence information of a cDNA fragment is used as a primer to obtain the 5' end region of the cDNA. For this purpose, the primer on the 3' side is designed based on the 'nucleotide sequence of the cDNA as close to 5' end as possible. The information of a cDNA fragment includes EST. Furthermore, information of a cDNA fragment is obtained *via* various gene analyses. Full-length nucleotide sequences are often determined based on information of such fragments. For example, when it is necessary to obtain the nucleotide sequence of 5' end of EST that is used as a probe for DNA array, the region of interest can be synthesized using a primer set of the present invention. cDNA fragments obtained by PCR cloning or such are sometimes used to obtain their full-lengths. Herein, the sequence information of a cDNA fragment can be defined as a primer having a nucleotide sequence complementary to a particular mRNA.

Furthermore, it is possible to use as the 3' primer a primer having the nucleotide sequence of a gene tag adjacent to the type II endonuclease recognition sequence in cDNA or the complementary sequence thereto. In SAGE as presently practice (SCIENCE, Vol.270, 484-487, Oct. 20, 1995), a region adjacent to a particular endonuclease site in cDNA is generated as a gene tag. Gene expression profiling can be performed based on the nucleotide sequence information of such tags. cDNA covering a substantial region of a gene of interest may be synthesized using as the 3' primer for the same analyte, the nucleotide sequence information of a gene tag selected by known analytical methods.

Among the primer sets described above, a combination including the oligo dT primer is particularly preferred as a primer set to synthesize full-length cDNA. Full-length cDNAs are useful in the mapping of transcriptional start sites. Determining at least the nucleotide sequence of a region that includes the 5' end is essential for identification of transcripts having different structures in the 5' UTR. Furthermore, it is generally believed to be difficult to obtain full-length cDNA. Under this circumstance, full-length cDNA synthesis, using gene tag information obtained according to the present invention, is especially useful. Specifically, the present invention relates to methods for synthesizing full-length cDNA, which include the following steps of:

(a) conducting complementary strand synthesis using RNA or cDNA as a template and using oligo dT primer as the 3' primer and a 5' primer for synthesizing cDNA that includes a nucleotide sequence, or the complementary sequence thereto, determined through the steps of:

(1) producing gene tags according to the method of the present invention; and
(2) determining the nucleotide sequences of gene tags of (1), and

(b) recovering the synthesized DNA as full-length cDNA.

cDNA is synthesized using, as a template, RNA obtained from cells that are likely to contain mRNA of interest and using the above-described primer set of the present invention. Alternatively, it is also possible to use as a template a cDNA library obtained from the cells. Those skilled in the art can synthesize cDNA based on given nucleotide sequence information of the primers. Specifically, cDNA of interest can be synthesized from RNA using known methods, such as RT-PCR. mRNA is preferably used as the RNA. When a cDNA library is used as a template, cDNA of interest can be synthesized by PCR. It is also possible to use commercially available cDNA libraries.

The present invention relates to full-length cDNA synthesized as described above. Herein, the full-length cDNA refers to cDNA having both poly(A) and nucleotide sequence information of the portion of the CAP structure of mRNA. The present invention also relates to a polypeptide encoded by full-length cDNA synthesized in accordance with the present invention. ORFs can be identified by analyzing the nucleotide sequence of full-length cDNA. Based on the identified ORF, the coding region can be introduced into expression vectors. The present invention includes expression vectors obtainable as described above. When such an expression vector is introduced into an appropriate expression system, the polypeptide encoded by the cDNA can be expressed and collected as a recombinant.

It is also possible, using *in vitro* translation, to express and collect as a recombinant a polypeptide encoded by the coding region of full-length cDNA of the present invention. Methods of *in vitro* translation are known. *In vitro* translation is also referred to as "cell-free protein translation". Specifically, translation into an amino acid sequence can be achieved by contacting a construct in which DNA encoding an amino acid sequence of interest has been operatively linked to a promoter, with an element supporting *in vitro* translation. Some transcriptional regulatory regions, such as a terminator, can be arranged in the construct. Such an element supporting *in vitro* translation is a mixture containing RNA polymerase, ribonucleotide substrates, amino acids, ribosome, tRNA, and the like. In the presence of these components necessary for protein translation, DNA can be translated into a protein without using cellular functions. RNA polymerase recognizes the above-described promoter and transcribes DNA as a template into mRNA under the control of the promoter. The ribonucleotide substrates ATP, GTP, CTP, and UTP are used in the transcription. The transcribed mRNA is translated into a polypeptide in the ribosome.

Commercially available *In vitro* translation kits can also be used as the element supporting *in vitro* translation. Cell-free protein translation kits, such as those using rabbit reticulocyte lysate (RRL), wheat germ extract (WGE), and *E. coli* lysate, are commercially available. A reconstituted *in vitro* transcription-translation system, using about 30 types of high-purity enzymes required for transcription, translation, and energy regeneration, has been previously established (Shimizu *et al.* (2001) Nature Biotechnology. vol.19, p.751-755) and is presently available as a kit.

The present invention also relates to antibodies that recognize such polypeptides. Such antibodies can be obtained, for example, by immunizing animals with the above-described recombinant or a domain peptide having an amino acid sequence selected from the translated amino acid sequence. Polyclonal antibodies can be collected from immunized animals. It is also possible to obtain monoclonal antibodies by cloning antibody-producing cells from immunized animals. Methods for screening for clones producing antibodies having a desired reactivity, which include preparing hybridomas through fusion of antibody-producing cells with cells of a cell line, such as myeloma, are known.

Brief Description of the Drawings

Fig. 1 is a schematic representation illustrating the method for obtaining gene tags according to the present invention. mRNA was divided into two equal portions and the CAP structure of mRNA was enzymatically replaced with either of two types of synthetic oligonucleotides containing *Mme*I, a type IIs restriction endonuclease, and *Xho*I restriction sites. Then, oligo-capping mRNA was converted into first strand cDNA with dT adapter primer. Second strand was synthesized with biotin-bound 5'-primer and dT adapter primer by PCR. The resulting double strand cDNA was cleaved with *Mme*I, which cleaves at a position 20 bp away from its recognition site. After 5'-cDNA was isolated by binding to streptavidin beads, the two pools of tags were ligated to each other.

Fig. 2 is a graph showing the distance of 5' SAGE tags relative to mRNA start sites in UniGene and DBTSS sequences. The distance is shown as the number of upstream (-) and downstream (+) nucleotides (x-axis). The mRNA start site in UniGene is depicted as 0. Frequency of 5' SAGE tag is given on the y-axis. A small distance between the aligned positions of each 5' SAGE tag and its corresponding gene implies that the 5'-tags are roughly consistent with known 5' transcriptional start site. The present inventors used UniGene and DBTSS databases separately to determine the difference of their coverage of transcriptional start sites.

Fig. 3 is a graph showing a scatter plot between frequencies of 5'-SAGE tags and 3' SAGE tags. The 5' SAGE and 3' SAGE tags hit to one locus in genome were analyzed as described in the section Materials and Methods of Example 2. In the figure, both axes are expressed in logarithm.

Exemplary Mode for Carrying out the Invention

The present invention is illustrated in detail below with reference to Examples.

[Example 1]

In this example, it was confirmed that gene tags including a nucleotide sequence from the 5' end of mRNA can be obtained by carrying out the experiment described below in accordance with the present invention. The procedure described below is summarized in Fig. 1.

Oligo-capping method

An oligo-capping method modified from the method of Maruyama and Sugano (Maruyama, K., Sugano, S., 1994. Oligo-capping: a simple method to replace the cap structure of eukaryotic mRNAs with oligoribo-nucleotides. Gene 138, 171-174.) was used. 5 to 10 μg of poly(A)+ RNA was treated with 1.2 unit of bacterial alkaline phosphatase (BAP; TaKaRa) in 100 μl of a mixture containing 100 mM Tris-HCl (pH 8.0), 5 mM 2-mercaptoethanol, and 100 units of RNasin (Promega) at 37°C for 40 minutes. After extraction with phenol:chloroform (1:1) twice and ethanol precipitation, the poly(A)+ RNA was treated with 20 units of tobacco acid pyrophosphatase (TAP) in 100 μl of a mixture containing 50 mM sodium acetate (pH 5.5), 1 mM EDTA, 5 mM 2-mercaptoethanol, and 100 units of RNasin at 37°C for 45 minutes.

After phenol:chloroform extraction and ethanol precipitation, 2 to 4 μg of the BAP-TAP treated poly(A)+ RNA was divided into two pools, and each pool was separately subjected to ligation with an RNA linker (either 5'-oligo 1 or 5'-oligo 2). 5'-oligo 1 and 5'-oligo 2 are RNAs composed of the nucleotide sequences shown below. Both RNA linkers have *Xho*I and *Mme*I recognition sequences.

5'-oligo 1/SEQ ID NO: 1

5'-UUU GGA UUU GCU GGU GCA GUA CAA CUA GGC UUA AUA CUC GAG UCC GAC - 3'

5'-oligo 2/SEQ ID NO: 2

5'-UUU CUG CUC GAA UUC AAG CUU CUA ACG AUG UAC GCU CGA GUC CGA C -3'

The RNA linkers were ligated at 20°C for 3 to 16 hours in 100 μl of a reaction mixture composed of 250 units of RNA ligase (TaKaRa), 100 units of Rnasin, and the following components:

50 mM Tris-HCl (pH 7.5)

5 mM $MgCl_2$,

5 mM 2-mercaptoethanol

0.5 mM ATP

25% PEG8000

cDNA synthesis

Two types of cDNA libraries were synthesized: a full-length cDNA-enriched library and a 5' end cDNA-enriched library. The full-length cDNA-enriched library is a library rich in full-length cDNA, containing cDNA synthesized from poly (A)+ mRNA as a template using oligo dT adapter primer. The 5' end cDNA-enriched library contains cDNA synthesized using a random adapter primer. By using the random adapter primer, cDNA is synthesized even from fragments lacking poly(A). Gene tags were obtained from each of these two types of cDNAs.

After removing unligated RNA linkers, cDNA was synthesized with RNaseH-free reverse transcriptase (Superscript II, Gibco BRL). For the full-length cDNA-enriched library, cDNA was synthesized using 10 pmol of dT adapter primer (SEQ ID NO: 3), which was added to 50 μl of a reaction mixture containing 2 to 4 μg of oligo-capped poly(A)+ RNA.

dT adapter primer (SEQ ID NO: 3)

5'-GCG GCT GAA GAC GGC CTA TGT GGC CTT TTT TTT TTT TTT TTT-3'

The reaction was conducted under the conditions recommended by the supplier (incubated at 42°C for one hour).

For the 5' end cDNA-enriched library, 10 pmol of random adapter primer (SEQ ID NO: 4) was used and incubated at 12°C for 1 hour and 42°C for another hour.

Random adapter primer (SEQ ID NO: 4)

5'-GCG GCT GAA GAC GGC CTA TGT GGC CNN NNN NC-3'

cDNA amplification

After first strand cDNA synthesis, RNA was degraded in 15 mM NaOH by incubating at 65°C for 1 hour. The cDNA that is made from 1 μg of oligo-capped poly(A)+ RNA as a template was amplified in a volume of 100 μl using an XL PCR kit (Perkin-Elmer) with 16 pmol of 5' PCR primer and 3' PCR primer (5'-GCG GCT GAA GAC GGC CTA TGT-3'/SEQ ID NO: 7). As the 5' PCR primer, the primers of SEQ ID NOs: 5 and 6 were used for the pools ligated with 5' oligo-1 and 5' oligo-2 RNA linkers, respectively.

5' PCR primer (SEQ ID NO: 5) for 5' oligo 1

5' biotin-GGA TTT GCT GGT GCA GTA CAA CTA GGC TTA ATA-3'

5' PCR primer (SEQ ID NO: 6) for 5'oligo 2

5' biotin-CTG CTC GAA TTC AAG CTT CTA ACG ATG TAC G-3'

3' PCR primer (SEQ ID NO: 7)

5'-GCG GCT GAA GAC GGC CTA TGT-3'

When a dT-adapter primer was used in the first strand synthesis, cDNA was amplified by 5 to 10 cycles of 94°C for one minute, 58°C for one minute, and 72°C for 10 minutes. When a random adapter primer was used in the first strand synthesis, cDNA was amplified by 10 cycles of 94°C for one minute, 58°C for one minute, and 72°C for 2 minutes.

PCR products were extracted with phenol:chloroform (1:1) once, ethanol-precipitated, and digested with the *Mme*I type IIs restriction endonuclease (University of Gdansk Center for Technology Transfer, Gdansk, Poland). Digestion was performed using 300 μl of a reaction mixture containing 10 mM HEPES (pH 8.0), 2.5 mM potassium acetate, 5 mM magnesium acetate, 2 mM DTT, 40 μM S-adenosylmethionine, and 40 units of *Mme*I at 37°C for 2.5 hours. The digested 5'-terminal cDNA fragments were bound to streptavidin-coated magnetic beads (Dynal, Oslo, Norway). To yield ditags, the cDNA fragments which bound to the beads were directly ligated together in 16 μl reaction containing 4 units T4 DNA ligase in the supplied buffer at 16°C for 2.5 hours.

The resulting ditags were amplified by PCR using the primers: 5'-GGA TTT GCT GGT GCA GTA CAA CTA GGC- 3' (SEQ ID NO: 8) and 5'-CTG CTC GAA TTC AAG CTT CTA ACG ATG-3' (SEQ ID NO: 9). The PCR products were analyzed by polyacrylamide gel electrophoresis (PAGE) and digested with *Xho*I. The band containing the ditags was excised and self-ligated to produce long concatemers. The concatemers were then cloned into the *Xho*I site of pZero 1.0 (Invitrogen).

Colonies were screened by PCR using M13 forward and M13 reverse primers. PCR products containing inserts of 600 bp or more were sequenced with the Big Dye terminator ver.3 and analyzed using a 3730 ABI automated DNA sequencer (Applied Biosystems, CA). All electropherograms were reanalyzed by visual inspection to check for ambiguous bases and to correct misreads.

The frequency of occurrence of each tag was determined using software prepared for this purpose. BLAST search (http://www.ncbi.nlm.nih.gov/BLAST/) and human genome database search (http://www.ncbi.nlm.nih.gov/genome/guide/human/) were performed using as queries the nucleotide sequences of tags obtained from the analysis.

Part of the analysis results for the nucleotide sequences of 3000 or more gene tags obtained from the 5' end cDNA-enriched library synthesized using the random adapter primer is summarized below. In the results shown below, the SEQ ID NO. of the nucleotide sequence of each gene tag is followed by the information indicated below being demarcated with "/". Following the information, information of a known gene that was hit by the query sequence is shown in a next new line (GenBank Accession No. and annotation).

- Nucleotide sequence of gene tag
- Frequency of occurrence of a gene tag relative to the total number of obtained gene tags
- Position of a known sequence to which the nucleotide sequence of gene tag hit (○: the hit sequence is expected to be at the 5' end; x: the hit sequence is expected not to be at the 5' end)

(SEQ ID NO: 10) / ACATCTGACCTCATGGAG / 27 / ○
gi|33694637|tpg|BK000408.1| TPA: Human adenovirus type 5, complete genome
(SEQ ID NO: 11) / CTCTTTCCTTGCCTAACG / 22 / ○
gi|17981705|ref|NM_001007.2| Homo sapiens ribosomal protein S4, X-linked (RPS4X), mRNA (SEQ ID NO: 12) / TACCTGGTTGATCCTGCC / 21 / x
(SEQ ID NO: 13) / CTTTTCCTGTGGCAGCAG / 20 / ○
<gi|16579884|ref|NM_000968.2| Homo sapiens ribosomal protein L4 (RPL4), mRNA
(SEQ ID NO: 14) / CTCTTCCGCCGTCGTCGC / 16 / ○
Homo sapiens eukaryotic translation elongation factor 2 (EEF2), upstream of mRNA
(SEQ ID NO: 15) / CTCATTGAACTCGCCTGC / 11 / ○
gi|28338|emb|X04098.1|HSACTCGR Homo sapiens mRNA for cytoskeletal gamma-actin (ACT G1 gene)
(SEQ ID NO: 16) / CTGGTTGATCCTGCCAGT / 11 / x
(SEQ ID NO: 17) / CTCAGTCGCCGCTGCCAG / 10 / ○
gi|28338|emb|X04098.1|HSACTCGR Homo sapiens mRNA for cytoskeletal gamma-actin (ACT G1 gene)
(SEQ ID NO: 18) / CTTTCACTGCAAGGCGGC / 10 / ○
gi|18314626|gb|BC021993.1| guanine nucleotide binding protein (G protein), beta polypeptide 2-1 ike 1
(SEQ ID NO: 19) / ACGCTGTGACAGCCACAC / 9 / ○
upstream ofNM_005382
(SEQ ID NO: 20) / GTGACAGCCACACGCCCC / 9 / x
gi|35045|emb|Y00067.1|HSNFM Human gene for neurofilament subunit M (NF-M)
(SEQ ID NO: 21) / AACGGCTAGCCTGAGGAG / 8 / x
gi|188487|gb|M59828.1|HUMMHHSP Human MHC class III HSP70-1 gene (HLA), complete cd s
(SEQ ID NO: 22) / AGTAGCAGCAGCGCCGGG / 8 / ○
gi|14043071|ref|NM_031243.1| Homo sapiens heterogeneous nuclear ribonucleoprotein A2/B 1
(SEQ ID NO: 23) / ATTCCTAGTTAAGGCGGC / 8 / ○
gi|5020073|gb|AF146651.1|AF146651 Homo sapiens glyoxalase-I gene, complete cds
(SEQ ID NO: 24) / AATTGTGTTCGCAGCCGC / 7 / ○
gi|22027640|ref|NM_002107.2| Homo sapiens H3 histone, family 3A (H3F3A), mRNA
(SEQ ID NO: 25) / ATATTTCTTACTCTCTCG / 7 / x
gi|37704377|ref|NR_001564.1| Homo sapiens X (inactive)-specific transcript (XIST) on chromos ome X
(SEQ ID NO: 26) / CTCAGTCGCCGCTGCCAA / 7 / ○
gi|28338|emb|X04098.1|HSACTCGR Homo sapiens mRNA for cytoskeletal gamma-actin
(SEQ ID NO: 27) / AAAACGGCCAGCCTGAGG / 6 / x gi|188489|gb|M59830.1|HUMMHHSP2 Human MHC class III HSP70-2 gene (HLA), complete c ds
(SEQ ID NO: 28) / CTCTCTTTCACTGCAAGG / 6 / ○
gi|12652914|gb|BC000214.1| guanine nucleotide binding protein (G protein), beta polypeptide 2-1 ike 1
(SEQ ID NO: 29) / AATTTCTACGCGCACCGG / 5 / ○
gi|402305|gb|L24369.1|HUMRPS4A Homo sapiens ribosomal protein S4 gene

(SEQ ID NO: 30) / ACCGCCGAGACCGCGTCC / 5 / ○
gi|10437878|dbj|AK025375.1| Homo sapiens ACTB mRNA for mutant beta-actin
(SEQ ID NO: 31) / AGACGCAGAGTAGATTGT / 5 / ○
gi|2315183|emb|Z82216.1|HS75N13 Human DNA sequence from clone RP1-75N13 on chromos ome Xq21.1,
(SEQ ID NO: 32) / AGTTCGATCGGTAGCGGG / 5 / x
gi|37540535|ref|XM_294582.2| Homo sapiens similar to DNA-binding protein B (LOC347295), mRNA
(SEQ ID NO: 33) / AGTTCTCGGGCGTACGGC / 5 / ○
gi|30581134|ref|NM_006306.2| Homo sapiens SMC1 structural maintenance of chromosomes 1-1 ike 1
(SEQ ID NO: 34) / AGTTGCTTCAGCGTCCCG / 5 / ○
gi|32487|emb|X15183.1|HSHSP90R Human mRNA for 90-kDa heat-shock protein
(SEQ ID NO: 35) / ATTAAACGGTTGCAGGCG / 5 / x
gi|33239450|ref|NM_182649.1| Homo sapiens proliferating cell nuclear antigen (PCNA)transcript variant 2, mRNA
(SEQ ID NO: 36) / CCGGCCGGGGGGCGGGCG / 5 / ○
gi|555853|gb|U13369.1|HSU13369 Human ribosomal DNA complete repeating unit
(SEQ ID NO: 37) / CCTTTTGGCTCTCTGACC / 5 / ○
gi|15718688|ref|NM_001006.2| Homo sapiens ribosomal protein S3A (RPS3A), mRNA
(SEQ ID NO: 38) / CTCAGTACAGCTCCGGCC / 5 / ○
gi|21217408|gb|AC015849.5| Homo sapiens chromosome 17, clone RP11-362K1, complete sequence
(SEQ ID NO: 39) / CTCTTTCGGCCGCGCTGG / 5 / o
gi|461248|dbj |D28421.1|HUMRPL80 Homo sapiens mRNA for ribosomal protein L8 homologue,
5'UTR

Of the obtained tags, the nucleotide sequences of 30 tags were analyzed, and the results showed that more than 73% (22/30) of the tags were actually derived from nucleotide sequences at the 5' ends of cDNAs. Accordingly, the nucleotide sequences from the 5' ends of mRNAs can be obtained as tags with high probability according to the present invention.

[Example 2]

Results obtained by gene expression analysis using gene tags including the nucleotide sequence from the 5' end of mRNA according to the present invention (hereinafter referred to as "5' SAGE") were compared with those obtained by conventional SAGE (hereinafter referred to as "3' SAGE").

Materials and Methods

Generation of 3'-Long SAGE library

Total RNA was isolated from HEK293 and mRNA was selected as previously described (Hashimoto, S.-i., Suzuki, T., Dong, H.-Y, Yamazaki, N. & Matsushima, K. Serial analysis of gene expression in human monocytes and macrophages. Blood 94, 837-844,1999). Long SAGE (Saha, S. *et al.* Using the transcriptome to annotate the genome. Nat Biotechnol 20, 508-512, 2002) was performed with 3 μg mRNA using the standard SAGE protocol with the following modifications.

After *Nla*III digestion, linker 1A (5'-TTT GGA TTT GCT GGT GCA GTA CAA CTA GGC TTA ATA TCC GAC ATG-3'/SEQ ID NO: 40) and linker 1B (5'-TCG GAT ATT AAG CCT AGT TGT ACT GCA CCA GCAAAT CC C7 amino modified-3'/SEQ ID NO: 41) were annealed together and ligated to half the cDNA population. Linker 2A (5'-TTT CTG CTC GAA TTC AAG CTT CTAACG ATG TAC GTC CGA CAT G-3'/SEQ ID NO: 42) and linker 2B (5'-TCG GAC GTA CAT CGT TAG AAG CTT GAA TTC GAG CAG C7 amino modified-3'/SEQ ID NO: 43) were annealed together and ligated to the remaining half of the cDNA. Thus, those linkers containing the *Mme*I recognition sequence were ligated to the 3' end of cDNA. Linker tag molecules were released from the cDNA using the *Mme*I type IIs restriction endonuclease (University of Gdansk Center for Technology Transfer, Gdansk, Poland). Digestion was performed using 40 units *Mme*I in 300 μl of a reaction mixture containing 10 mM HEPES (pH 8.0), 2.5 mM potassium acetate, 5 mM magnesium acetate, 2 mM DTT, and 40 μM S-adenosylmethionine at 37°C for 2.5 hours. The linker 1 tag and linker tag 2 molecules were directly ligated together in 16 μl reaction containing 4 units T4 DNA ligase in the supplied buffer at 16°C for 2.5 hours.

The released tags were ligated to one another, concatenated, and cloned into the *Sph*I site of pZero 1.0 (Invitrogen). Colonies were screened by polymerase chain reaction (PCR) using M13 forward and M13 reverse primers. PCR products containing inserts of 600-bp or longer were sequenced with the Big Dye terminator ver. 2 and analyzed using a 3730 ABI automated DNA sequencer (Applied Biosystems, CA). All electropherograms were reanalyzed by visual inspection to check for ambiguous bases and to correct misreads. SAGE 2000 software (version 4.12) was used to quantify the abundance of each tag. After elimination of linker sequences, other potential artifacts, and the repeated ditags, each tag was analyzed.

Generation of 5'-SAGE library

Oligo-capping was performed as described by Maruyama and Sugano (Maruyama, K. & Sugano, S. Oligo-capping: a simple method to replace the cap structure of eukaryotic mRNAs with oligoribonucleotides. Gene 138, 171-174, 1994) with some modifications (Suzuki, Y, Yoshitomo-Nakagawa, K., Maruyama, K., Suyama, A. & Sugano, S. Construction and characterization of a full length-enriched and a 5'-end-enriched cDNAlibrary. Gene 200, 149-156, 1997).

Specifically, 5 to 10 μg of poly(A)+ RNA was treated with 1.2 units of bacterial alkaline phosphatase (BAP; TaKaRa) in 100 μl of a reaction mixture containing 100 mM Tris-HCl (pH 8.0), 5 mM 2-mercaptoethanol, and 100 units of RNasin (Promega) at 37˚C for 40 minutes. After extraction with phenol: chloroform (1:1) twice and ethanol precipitation, the poly(A)+ RNA was treated with 20 units of tobacco acid pyrophosphatase (TAP) in 100 μl of a reaction mixture containing 50 mM sodium acetate (pH 5.5), 1 mM EDTA, 5 mM 2-mercaptoethanol, and 100 units of RNasin at 37˚C for 45 minutes. After phenol:chloroform extraction and ethanol precipitation, 2 to 4 μg of BAP-TAP treated poly(A)+ RNA was divided into two pools, and each pool was ligated with one of the following RNA linkers containing *Xho*I and *Mme*I recognition sites: 5'-oligo 1 (5'-UUU GGA UUU GCU GGU GCA GUA CAA CUA GGC UUA AUA CUC GAG UCC GAC -3'/SEQ ID NO: 1) and 5'-oligo 2 (5'-UUU CUG CUC GAA UUC AAG CUU CUA ACG AUG UAC GCU CGA GUC CGA C -3'/SEQ ID NO: 2). The RNA linkers were ligated using 250 units of RNA ligase (Takara) in 100 ml of a reaction mixture containing 50 mM Tris-HCl (pH 7.5), 5 mM $MgCl_2$, 5 mM 2-mercaptoethanol, 0.5 mM ATP, 25% PEG8000, and 100 units of RNasin at 20˚C for 3 to 16 hours.

After removing unligated 5'-oligo, cDNA was synthesized with RNaseH-free reverse transcriptase (Superscript II; Gibco BRL). For the 5' end-enriched cDNA library, 10 pmol of random adapter primer (5'-GCG GCT GAA GAC GGC CTA TGT GGC CNN NNN NC-3'/SEQ ID NO: 4) was used and incubated at 12˚C for 1 hour and 42˚C for another hour.

After first strand synthesis, RNA was degraded in 15 mM NaOH by incubating at 65˚C for 1 hour. The cDNA that is made from 1 mg of oligo-capped poly(A)+ RNA was amplified in a volume of 100 μl using an XL PCR kit (Perkin-Elmer) with 16 pmol of 5' (5' biotin-GGA TTT GCT GGT GCA GTA CAA CTA GGC TTAATA-3'/SEQ ID NO: 5, or 5' biotin-CTG CTC GAA TTC AAG CTT CTA ACG ATG TAC G-3'/SEQ ID NO: 6) and 3' (5'-GCG GCT GAA GAC GGC CTA TGT-3'/SEQ ID NO: 7) PCR primers. The cDNA prepared through extension using random adapter primer was amplified by 10 cycles of: 94˚C for one minute, 58˚C for one minute, and 72˚C for 2 minutes. PCR products were extracted with phenol:chloroform (1:1) once, ethanol precipitated, and digested with the *Mme*I type IIS restriction endonuclease (University of Gdansk, Center for Technology Transfer, Gdansk, Poland). Digestion was performed using 40 units *Mme*I in 300 μl of a reaction mixture containing 10 mM HEPES (pH 8.0), 2.5 mM potassium acetate, 5 mM magnesium acetate, 2 mM DTT, and 40 μM S-adenosylmethionine at 37˚C for 2.5 hours.

The digested 5'-terminal cDNA fragments were bound to streptavidin-coated magnetic beads (Dynal, Oslo, Norway). cDNA fragments which bound to the beads were directly ligated together in 16 μl reaction containing 4 units T4 DNA ligase in the supplied buffer at 16˚C for 2.5 hours. The ditags were amplified by PCR using the following primers: 5'-GGA TTT GCT GGT GCA GTA CAA CTA GGC -3'/SEQ ID NO: 8 and 5'-CTG CTC GAA TTC AAG CTT CTA ACG ATG-3'/SEQ ID NO: 9. The PCR products were analyzed by polyacrylamide gel electrophoresis (PAGE) and digested with *XhoI.* The band containing the ditags was excised and self-ligated to produce long concatemers. The concatemers were cloned into the *Xho*I site of pZero 1.0 (Invitrogen). Colonies were screened by PCR using M13 forward and M13 reverse primers. PCR products containing inserts of 600 bp or longer were sequenced with the Big Dye terminator ver. 3 and analyzed using a 3730 ABI automated DNA sequencer (Applied Biosystems, CA). All electropherograms were reanalyzed by visual inspection to check for ambiguous bases and to correct misreads. SAGE 2000 software (version 4.12) was used to quantify the abundance of each tag.

Association of 5'SAGE tags with corresponding genes

To assess the validity of 5'SAGE tags for identifying transcriptional start sites, the present inventors avoided aligning 5' SAGE tags with current cDNA/EST database, because the sequences are not always read from their transcriptional start sites. Instead, 5' tags obtained by the inventors were aligned with the human genome sequence (NCBI build 34) available from UCSC Genome Bioinformatics (http://genome.ucsc.edu/), by using the alignment program ALPS that is publicized by the University of Tokyo (http://alps.gi.k.u-tokyo.ac.jp/). Tags that matched in the sense orientation were only considered for this analysis.

Subsequently, neighborhood of the alignment location of each 5'-tag was searched to find its corresponding transcript by utilizing the Gene Resource Locator database (Honkura, T., Ogasawara, J., Yamada, T. & Morishita, S. The Gene Resource Locator: gene locus maps for transcriptome analysis. Nucleic Acids Res. 30, 221-225, 2002 URL http://grl.gi.k.u-tokyo.ac.jp/), a database of alignments of sequences in various resources such as UniGene (Build 162) (Wheeler, D.L. Database Resources of the National Center for Biotechnology. Nucleic Acids Res. 31, 28-33, 2003 URL ftp://ftp.ncbi.nih.gov/repository/UniGene/). The major problem was that, due to retrotransposition and genome-duplication, one 5'-tag could be aligned with multiple locations, though many of them were non-coding regions. This issue was resolved by selecting gene coding locations that were annotated in the UniGene database. Although 3'-tags often fell in the 3'-end exons, 5'-tags did not necessarily hit the first exons. Thus the search was made within 500 bp distance from the alignment position of each 5'-tag.

Consistency with known 5' transcriptional start site

A small distance between the aligned positions of each 5' SAGE tag and its corresponding gene implies that the 5'-tag is roughly consistent with the known 5' transcriptional start site. To calculate the distance, however, it should be noted that, near the 5'-tag, multiple cDNA/EST sequence alignments may be frequently observed as a result of alternative splicing. To resolve this issue and assign a unique value to the distance, an alignment that is closest to the 5' tag was selected. The distance was defined as negative if the 5'-tag was located in the upstream region of its corresponding cDNA. Otherwise, the value was defined positive or zero. In particular, a zero distance indicates perfect coincidence. To determine the overall distance distribution, the total number of 5'SAGE tag occurrences of the -500 to +200 nt of mRNA start sites was calculated. RefSeq, UniGene (GRL), and DBTSS databases were separately used to see the difference of their coverage of transcriptional start sites.

Results

5' SAGE

To obtain comprehensive information for transcriptional start sites, the present inventors developed the 5' SAGE using oligo-capping method. The 5' SAGE method generates 19 to 20 bp tags derived from the 5' ends of transcripts that can rapidly be analyzed and matched to genome sequence data. Fig. 1 shows the strategy associated with the 5' SAGE method.

Genome mapping

Using this method, the present inventors characterized 25,684 transcripts expressed in HEK293 cells as a test cell line and compared these with human genome sequence. A total of 19,893 tags perfectly matched to genomic sequences representing 13,404 different tags (Table 1).

80% (10,706 tags) of 13,404 different tags were assigned to unique positions. The tags matching multiple sites in the genome were as follows: 11.1% (1483 tags) to two loci, 8.1% (1090 tags) to 3-99 loci, and 0.9% (125 tags) to 100 or more loci. The tags mapped to multiple genomic loci corresponded mostly to retrotransposon elements, repetitive sequences, or pseudogenes.

Table 1

| Tag loci in genome # | 5'-end SAGE tag to genome# | | | 3'-end SAGE tag to genome## | | |
|---|---|---|---|---|---|---|
| | Tags mapped to genome (%) | Unique Tags mapped to genome(%) | Relative expression level | Tags mapped to genome (%) | Unique Tags mapped to genome (%) | Relative expression level |
| 1 loci/ genome | 15,448 (77.7) | 10,706 (79.9) | 1.44 | 34,139 (63.2) | 11,613 (75.3) | 2.94 |
| 2 loci/ genome | 2,037(10.2) | 1,483(11.1) | 1.37 | 6,739 (12.5) | 1,395(9.0) | 4.83 |
| 3~99 loci/ genome | 2,275(11.4) | 1,090 (8.1) | 2.09 | 12,265 (22.7) | 2,039 (13.2) | 6.02 |
| >100 loci/ genome | 133 (0.7) | 125 (0.9) | 1.06 | 907(1.7) | 376 (2.4) | 2.42 |

(continued)

| Tag loci in genome # | 5'-end SAGE tag to genome# | | | 3'-end SAGE tag to genome## | | |
|---|---|---|---|---|---|---|
| | Tags mapped to genome (%) | Unique Tags mapped to genome(%) | Relative expression level | Tags mapped to genome (%) | Unique Tags mapped to genome (%) | Relative expression level |
| Total tag | 19,893 (100) | 13,404 (100) | 1.40 | 54,050 (100) | 15,422 (100) | 2.13 |

Table 1: Experimental matching of SAGE tag to genome

#: Number of tags hit to genome using 18-bp 5'SAGE tags. Mapping was performed as descrived in Materials and Methods. 5,791 of the 25,684 tags sequenced did not match to genome. Relative expression level was determined by dividing the total number of transcript tags observed in the library by the number of different tags.

##: Number of tags hit to genome using 20-bp 3'SAGE tags. Mapping was performed as described in Materials and Methods. 27,162 of 81,211 tags sequenced did not match to genome.

Mapping to mRNA start site

Next, the present inventors calculated whether the 5' SAGE tags match the mRNA start sites. The present inventors used three databases, including the reference sequence database (RefSeq), the Gene Resource Locator (GRL) database which assembles gene maps that include information on cis-elements in regulatory regions and alternatively spliced transcripts, and the DataBase of Transcriptional Start Site (DBTSS; Suzuki, Y. *et al.* DBTSS: DataBase of human Transcriptional Start Sites and full-length cDNAs. Nucleic Acids Res 30, 328-331, 2002) which contains systematic 5' end sequences of human full-length cDNAs. Fig. 2 displays distance distributions, and Table 2 presents the number and the ratio of tag occurrences of a small distance, indicating that the 5' SAGE tags obtained by the present inventors coincide well with start site information of each database. 85.8 to 98.2% of tags mapped to each database were assigned within -500 to +200 nucleotides of mRNA start sites.

Notably, 23.5 to 49.3% of 5' SAGE tags hit the upstream regions of the defined transcription start sites (TSS) in these databases. Moreover, the present inventors examined the nucleotide preference at the TSS by 5' SAGE tags. It has been reported that the nucleotides of the TSS were A (47%), G (28%), C (14%), and T (12%) using 5880 mRNAs in 276 human genes (Suzuki, Y *et al.* Diverse transcriptional initiation revealed by fine, large-scale mapping of mRNA start sites. EMBO Rep 2, 388-393, 2001). The data obtained by the present inventors also showed very similar percentage of the use of the first nucleotide: A (41 %), G (32%), C (17%), and T (10%). Taken together, the 5' SAGE tag method of the present inventors can precisely identify the TSS. The data provides the present inventors not only with accurate transcriptional start site information but also a resource for analyzing promoter usage. Interestingly, 33% of total sequenced tags in 5' SAGE in this study did not match to genome matching. Among them, 39% of first nucleotide of 5' SAGE tags unmatched to genome was also A. Some of tags that did not match to genome can be considered to hit the regions with single nucleotide mutation or a deletion in genome.

Table 2

| Distance from start site of each database (nt) | Tag number (%) | | |
|---|---|---|---|
| | RefSeq | UniGene (GRL) | DBTSS |
| -500 ~ -201 | 349 (3.2) | 204 (1.5) | 160 (1.6) |
| -200 ~ -51 | 887 (8.1) | 335 (2.4) | 253 (2.5) |
| -50 ~ -1 | 4,179 (38.1) | 3,957 (28.8) | 1,965 (19.5) |
| 0 ~ +50 | 3,173 (28.9) | 8,673 (63.2) | 7,149 (70.8) |
| +51 ~ +200 | 837 (7.6) | 311 (2.3) | 209 (2.1) |
| (-500~ +200) | 9,425 (85.8) | 13,480 (98.2) | 9,736 (96.4) |
| Total tags | 10,982 (100) | 13,723 (100) | 10,098 (100) |

Table 2: Distance of 5'SAGE tags relative to mRNA start sites in each database

The tags whose correspondence in mapping with the 5' ends of genes from each database were analyzed as described in Fig. 2.

Identification of novel genes or unannotated genes

In order to identify the uncharacterized genes, 5' SAGE tags were compared with the genome sequence, RefSeq, and EST databases. Of the 10,706 unique tags with single locus in the genome, 9,376 tags were associated with their corresponding UniGene ESTs (Table 3). Furthermore, 6,418 unique 5' SAGE tags were associated with known genes in DBTSS. The remaining tags (12.4%) matched the regions within intron (5.4%) of known genes or uncharacterized regions (6.6%). Tags matching uncharacterized regions hit mainly to two sites:

(1) completely uncharacterized regions; and
(2) regions of uncharacterized EST.

The evidence of the expression of such genes enables the discovery of the novel genes in their full-length form by referring 3'SAGE.

Table 3

| | UniqueTags mapped to genome (tags occurrences) | |
|---|---|---|
| Gene/exon category | 5'SAGE | 3' SAGE |
| **Previously annotated** | | |
| Known genes | 9,376 (13,674) | 8,359 (27,996) |
| **Previously unannotated** | | |
| Internal exons (Intron) | 515 (713) | 1,329 (2,442) |
| genome | 815 (1,061) | 1,925 (3,701) |
| Total | 10,706(15,448) | 11,613(34,139) |

Table 3: Identification of uncharacterized candidate genes and exons

10,706 tags were assigned to unique positions, and 9,376 tags were associated with their corresponding UniGene ESTs.

SAGE is a very powerful method that can be used to obtain quantitative information on the abundance of transcripts. Table 4 shows the 5'-end of the transcripts profiled in HEK 293 cells. The most expressed genes were identified as neurofilament 3 (NEF3), with an expression frequency of 1.43%, followed by genes that hit to multiple loci and the elongation factor 2. Several genes, such as NEF3, heat shock 70 kDa protein 1A, calreticulin, and heterogeneous nuclear ribonucleoprotein H1, represented different tags. This suggests that several genes were transcribed from different TSSs. For example, heat shock 70 kDa protein 1A is transcribed from eight different transcriptional start sites, and calreticulin is transcribed from seven different transcriptional start sites. These results suggest that individual transcriptional start sites are associated with gene expression. Some of nucleotide sequences shown in Table 4 are also described in the results of Example 1 described above. Table 4 also includes results obtained by comparing the obtained gene tag sequences with genomic sequences, while in Example 1 the gene tag sequences were not compared with genomic sequences. Thus, even when the nucleotide sequence of a gene tag is identical, the description in the column "Gene" of Table 4 may vary from the annotation described in Example 1.

Table 4

| Tag sequence | SEQ ID NO. | Tag count | Related Unigene cluster | Related refseq | Gene |
|---|---|---|---|---|---|
| GCTGTGACAGCC ACACGC | 44 | 286 | Hs.71346 | NM_005382 | Homo sapiens neurofilament 3 (150kDa medium) (NEF3), mRNA |
| CTTTTCCTGTGG CAGCAG | 13 | 171 | | | Multiple hit to genome |
| CTCTTTCCTTGC CTAACG | 11 | 127 | | | Multiple hit to genome |

(continued)

| Tag sequence | SEQ ID NO. | Tag count | Related Unigene cluster | Related refseq | Gene |
|---|---|---|---|---|---|
| CTCTTCCGCCGT CGTCGC | 14 | 120 | Hs.75309 | NM_001961 | eukaryotic translation elongation factor 2 |
| TACCTGGTTGAT CCTGCC | 12 | 117 | | | Multiple hit to genome |
| CTGGTTGATCCT GCCAGT | 16 | 89 | | | Multiple hit to genome |
| AACGGCTAGCCT GAGGAG | 21 | 83 | Hs.274402,H s. 75452,Hs.8 0288 | NM_ 005345,NM 005346 | heat shock 70kDa protein 1A |
| AGTAGCAGCAGC GCCGGG | 22 | 75 | Hs.232400 | NM_ 031243,NM _002137 | heterogeneous nuclear ribonucleoprotein A2/B1 |
| CTCATTGAACTC GCCTGC | 15 | 68 | | | Multiple hit to genome |
| GTGACAGCCACA CGCCCC | 20 | 66 | Hs.71346 | NM_005382 | Homo sapiens neurofilament 3 (150kDa medium) (NEF3), mRNA |
| AGTTCGATCGGT AGCGGG | 32 | 57 | | | Multiple hit to genome |
| ACGCTGTGACAG CCACAC | 19 | 56 | Hs.71346 | NM_005382 | Homo sapiens neurofilament 3 (150kDa medium) (NEF3), mRNA |
| CTTTTTCGCAAC GGGTTT | 45 | 55 | | | Multiple hit to genome |
| AATTTCTACGCG CACCGG | 29 | 54 | Hs.446628 | NM_001007 | ribosomal protein S4, X-linked |
| ACCGCCGAGACC GCGTCC | 30 | 53 | Hs.426930,H s. 510444 | NM_001101 | actin, beta |
| CTTTCACTGCAA GGCGGC | 18 | 52 | Hs.5662,Hs. 509234 | NM_006098 | guanine nucleotide binding protein (G protein), beta polypeptide 2-like 1 |
| ATATTTCTTACT CTCTCG | 25 | 48 | | | Homo sapiens X (inactive)-specific transcript (XIST) on chromosome X |

(continued)

| Tag sequence | SEQ ID NO. | Tag count | Related Unigene cluster | Related refseq | Gene |
|---|---|---|---|---|---|
| CTCAGTCGCCGC TGCCAG | 17 | 43 | Hs.14376,Hs . 500737 | | actin, gamma 1 |
| ATTCCTAGTTAA GGCGGC | 23 | 42 | Hs.268849 | NM_006708 | glyoxalase I |
| AATTGTGTTCGC AGCCGC | 24 | 37 | | | Multiple hit to genome |
| CCTCCTCATCAC ACGCCG | 46 | 37 | Hs.15589 | NM_004774 | PPAR binding protein |
| CTTTCTGCCCGT GGACGC | 47 | 37 | | | Multiple hit to genome |
| AGTACAGCTCCG GCCGCC | 48 | 35 | Hs.402752 | NM _003487,NM _139215 | TAF15 RNA polymerase II, TATA box binding protein (TBP)-associated factor, 68kDa |
| CACCTGTTTGCA GGCTGC | 49 | 34 | Hs.146550 | | myosin, heavy polypeptide 9, non-muscle |
| CTCTCTTTCACT GCAAGG | 28 | 33 | Hs.5662,Hs. 509234 | NM_006098 | guanine nucleotide binding protein (G protein), beta polypeptide 2-like 1 |
| AGTTGCTTCAGC GTCCCG | 34 | 30 | Hs.446579,H s. 449634 | NM_005348 | heat shock 90kDa protein 1, alpha |
| AGTTCTCGGGCG TACGGC | 33 | 29 | Hs.211602 | NM_006306 | SMC1 structural maintenance of chromosomes 1-like 1 (yeast) |
| GTCCGTACTGCA GAGCCG | 50 | 29 | Hs.353170 | NM_004343 | calreticulin |
| AAAACGGCCAGC CTGAGG | 27 | 27 | Hs.75452 | | FLJ38698 |
| ATTTCGTCTTAG CCACGC | 51 | 26 | Hs.202166 | | heterogeneous nuclear ribonucleoprotein H1 (H) |
| AGGCATTGAGGC AGCCAG | 52 | 25 | | | hit to genome |
| AGTGGGCGGACC GCGCGG | 53 | 25 | Hs.192374 | NM_003299 | tumor rejection antigen (gp96) 1 |

(continued)

| Tag sequence | SEQ ID NO. | Tag count | Related Unigene cluster | Related refseq | Gene |
|---|---|---|---|---|---|
| CCCAATTTCTAC GCGCAC | 54 | 25 | Hs.446628 | NM_001007 | ribosomal protein S4, X-linked |
| CTCGTTGCGCAG TAGTGC | 55 | 25 | Hs.380118,H s. 460941 | | RNA binding motif protein, X-linked |
| GTGCTGCAGCCG CTGCCG | 56 | 25 | Hs.2795 | NM_005566 | lactate dehydrogenase A |
| CATTTCGTCTTA GCCACG | 57 | 24 | Hs.202166 | | heterogeneous nuclear ribonucleoprotein H1 (H) |
| CTCTTTCCCTAA GCAGCC | 58 | 24 | | | Multiple hit to genome |
| GACTAATTTGTT GGCGGC | 59 | 24 | Hs.280311 | | myosin, heavy polypeptide 10, non-muscle |
| ACCTCATTCATT TCTACC | 60 | 23 | Hs.279806 | NM_004396 | DEAD (Asp- Glu-Ala-Asp) box polypeptide 5 |
| CCTTTCTGCCCG TGGACG | 61 | 23 | | | Multiple hit to genome |
| AGTATCTGTGGG TACCCG | 62 | 22 | Hs.433455,H s. 331035 | NM_001428 | enolase 1, (alpha) |
| CAATTTCTACGC GCACCG | 63 | 22 | Hs.446628 | NM_001007 | ribosomal protein S4, X-linked |
| GCACACAGCCAT CCATCC | 64 | 22 | Hs.107600 | NM_006158 | neurofilament , light polypeptide 68kDa |
| AGTGACGCGTAT TGCCTG | 65 | 19 | Hs.75337,Hs . 467172 | NM_004741 | nucleolar and coiled-body phosphoprotein 1 |
| CTCTTTCCAGCC AGCGCC | 66 | 19 | | | Multiple hit to genome |
| CTTTTCCGCCCG CTCCCC | 67 | 19 | Hs.374596 | NM_003295 | tumor protein, translationally -controlled 1 |
| GCGTCTTGTTCT TGCCTG | 68 | 19 | Hs.180909 | NM_ 181696,NM _181697,NM_ 00 2574 | peroxiredoxin 1 |
| ATATAGAGGCTG GGGGTG | 69 | 18 | Hs.427152 | | high density lipoprotein binding protein (vigilin) |

(continued)

| Tag sequence | SEQ ID NO. | Tag count | Related Unigene cluster | Related refseq | Gene |
|---|---|---|---|---|---|
| ATTAAACGGTTG CAGGCG | 35 | 18 | Hs.78996,Hs . 449476 | NM_002592 | Proliferating cell nuclear antigen |
| CCTTTTGGCTCT CTGACC | 37 | 18 | | | Multiple hit to genome |

Table 4: 5' end of transcription profile in HEK293 cells

The top fifty 5'-end transcripts expressed in HEK293 cells are listed herein. The tag sequences represent the 18-bp SAGE tag. Tags and their corresponding Unigene/ESTs are listed.

Correspondence between 5' and 3' SAGE tag expression

To validate the accuracy of 5' SAGE, the present inventors also performed 3'-Long SAGE for mRNA in the same cells. In 3'-Long SAGE, the present inventors characterized 81,212 transcript tags expressed in HEK293 cell line. A total of 54,050 tags matched genomic sequences representing 15,423 different tags (Table 1). 75% (11,613 tags) of 15,423 different tags matched one site in genome. Furthermore, 8,359 types of 3' SAGE tags were associated with known genes in UniGene EST (Table 3). The tags matching multiple sites in the genome were as follows: 9% (1395 tags) to two loci, 13.2% (2,039 tags) to 3-99 loci, and t 2.4% (376 tags) to 100 or more loci. The percentage of tags that matched multiple sites in the genome was very similar between 5' SAGE and 3' SAGE (Table 2). On the other hand, 5' SAGE tags were very heterogeneous as compared with 3' SAGE tags.

Saha *et al.* have also shown that tags present at more than 10 copies per genome are more highly expressed on average than those present at only one copy per genome (Saha, S. *et al.* Nat Biotechnol 20, 508-512, 2002). The data obtained by the present inventors also demonstrated that the relative expression level was higher in 3 to 99 loci/ genome than other fractions in the 5' SAGE and 3' SAGE libraries. This is due to the mechanism of correlation between gene expression and gene replication through retrotransposition. To estimate the extent of similarity between two libraries, the comparison of the expressed genes between 5' SAGE and 3'-Long SAGE was performed.

Since the 5' and 3' tags are randomly sampled from the 5' and 3' ends separately, the probability that the 5' tags are associated with a particular full-length cDNA sequence is expected to coincide with the probability that the 3' tags are matched to the cDNA,. However, due to the incomplete collection of full-length cDNA sequences or alternatively spliced transcripts, it is not straightforward to determine the exact correspondence between the 5' and 3' tags even though these tags might be originated from the same coding region. One promising approach would be to put together EST alignments that share exons in common, treat such a cluster as a gene coding locus, map the 5' and 3' SAGE tags to these clusters and their upstream regions, and uncover a correspondence between 5' and 3' SAGE tag expression. In this way, the present inventors counted pairs of 3' (horizontal axis) and 5' (vertical axis) tag occurrence numbers for each gene coding region, and Fig. 3 presents all the pairs in two-dimensional plane. Comparison of the expression patterns revealed that most genes were expressed at similar levels between both libraries. However, several transcripts were expressed at significantly different levels, and Pearson correlation coefficients of 5' and 3' SAGE libraries showed moderate similarity, at 0.36.

The reason for the moderate correlation is due to the dispersion of frequency from the 5' and 3' SAGE libraries. There are several possibilities for appearance of these tags, such as sequences derived from:

(1) a PCR amplification error in 5' SAGE and 3' SAGE;
(2) a small number of genes that would be expected to appropriate the *Nla*III restriction site in 3' SAGE;
(3) a small number of genes that would be expected to appropriate the *Xho*I restriction sites in 5' SAGE;
(4) an unknown splicing variant of mRNA in 5' SAGE and 3' SAGE; and
(5) an annotation error for tags matched to multiple genomic loci or EST annotated error into genome.

This study identified only a fraction of the genes expressed in HEK 293 cells as an example. A much larger number of tags, from a variety of different cell types and environmental conditions, would be required to thoroughly describe the compendium of expressed genes. Accumulation of data may resolve the problem of correspondence between 5' and 3' SAGE tag expression.

Discussion

Several groups have reported that mRNA start sites (Suzuki, Y. *et al.* Diverse transcriptional initiation revealed by fine, large-scale mapping of mRNA start sites. EMBO Rep 2, 388-393, 2001) and polyadenylation cleavage sites (Pauws, E., van Kampen, A.H., van de Graaf, S.A., de Vijlder, J.J. & Ris-Stalpers, C. Heterogeneity in polyadenylation cleavage sites in mammalian mRNA sequences: implications for SAGE analysis. Nucleic Acids Res 29,1690-1694, 2001) show heterogeneity. Although Shiraki *et al.* reported the difference of TSS of the specific gene during an organization (Shiraki, T. *et al.* Cap analysis gene expression for high-throughput analysis of transcriptional starting point and identification of promoter usage. Proc Natl Acad Sci USA 100, 15776-15781, 2003), the data of the present inventors show that the diversity of TSS already exists in a cell. Moreover, the data of the present inventors provide direct evidence for the heterogeneity of TSS and the 3' end region by means of the 5' SAGE and 3' SAGE methods.

For instance, the PPAR binding protein has one TSS and two 3' SAGE tag sites; ribosomal protein S4 has 16 TSSs and one 3' SAGE tag site; and calreticulin has seven TSSs and one 3' SAGE tag site. In addition, alternative mRNA splicing is a pivotal contribution to the complexity of the human proteome. Recent genome studies have demonstrated that 40 to 60% of human genes are alternatively spliced (Modrek, B. & Lee, C. A genomic view of alternative splicing. Nature Genetics 30, 13-19, 2002). It has been estimated that 15% of point mutations cause human genetic diseases by a mRNA splicing defect (Krawczak, M., Reiss, J. & Cooper, D.N. The mutational spectrum of single base-pair substitutions in mRNA splice junctions of human genes: causes and consequences. Hum Genet 90, 41-54, 1992).

Zavolan *et al.* have reported that among the transcription units with multiple splice forms, 49% contain transcripts in which usage of an alternative transcription start is accompanied by alternative splicing of the initial exon (Zavolan, M. *et al.* Impact of alternative initiation, splicing, and termination on the diversity of the mRNA transcripts encoded by the mouse transcriptome. Genome Res 13, 1290-1300, 2003). The present inventors also found that each mRNA start site of several genes, such as peroxiredoxin 4 (NM_006406), represents not only a different splicing variant of mRNA but also a different amount of gene expression. This implies that alternative transcription may frequently induce alternative splicing.

Recently, a new method to identify the transcriptional starting point using a cap-trapper system has been reported (Shiraki, T. *et al.* Proc Natl Acad Sci USA 100, 15776-15781, 2003). However, the efficiency of identification of mapping mRNA start site is not prominent by this method. The 5' SAGE method described in the study of the present inventors facilitates an exact mapping of the TSS and also establishes the frequency of gene expression.

In conclusion, the use of 5' SAGE method can considerably facilitate the annotation of genomes. Since 5' SAGE represents one of the few high throughput discovery approaches that does not depend on an *a priori* knowledge of gene sequences, such data will immediately allow independent validation of *in silico* gene predictions and identification of unannotated regions. In addition, the 5' SAGE method will be useful for finding SNPs in 5'UTR/promoter regions. Comprehensive identification of the gene transcribed from specific mRNA start sites in different types not only provides novel insight into the explanation of functional complexity of the human genome but also the diagnostic basis for various disorders such as cancer, diseases of the immune system and neurological diseases.

Finally, taking consideration of the diversity of the 5' end, it would be more appropriate to perform 5' SAGE than 3' SAGE for the determination of the frequency of gene expression.

Industrial Applicability

The present invention is useful for obtaining gene tags. A gene tag is a nucleotide sequence that is specific to a gene. Thus, it is thought that the frequency of occurrence of a tag in a gene library reflects expression levels of all genes constituting the library. Gene tags are thus useful in gene expression analysis. In particular, gene tags obtained in accordance with the present invention are generated based on the 5' end structure shared by all mRNA. Thus, results of gene expression analysis using tags that are generated according to the present invention are more reliable.

Tags of the present invention include nucleotide sequence information from the 5' end region of mRNA. Thus, transcriptional start sites in the genome can be identified based on the nucleotide sequence information of tags generated in accordance with the present invention. Furthermore, oligonucleotides designed based on the nucleotide sequence information of tags of the present invention can be used as primers for full-length cDNA synthesis.

All the prior art documents cited herein are incorporated herein by reference.

## Claims

1. A method for producing a gene tag for eukaryotic cells, which comprises the steps of:

(1) linking an RNA linker, comprising a type IIs endonuclease recognition sequence, to the CAP site of an RNA;
(2) synthesizing a cDNA using the resulting RNA of (1) as a template; and
(3) reacting the resulting cDNA of (2) with a type IIs endonuclease that recognizes the recognition sequence in the RNA linker, and thereby generating the gene tag.

2. The method of claim 1, wherein step (2) comprises synthesizing a cDNA by the steps of:

(i) synthesizing a first strand of the cDNA using a primer that anneals to an arbitrary region of the RNA; and
(ii) preparing a double-stranded cDNA by synthesizing a second strand of the cDNA using a primer that anneals to a region of the first strand which has been synthesized using the RNA linker as a template.

3. The method of claim 2, wherein the primer that anneals to a region of the first strand which has been synthesized using the RNA linker as a template has a label that binds to a solid phase or is immobilized onto a solid phase, wherein the method further comprises the step of recovering the double-stranded cDNA by recovering the solid phase.

4. The method of claim 3, wherein the solid phase is recovered before or after reaction with the type IIs endonuclease.

5. The method of claim 1, wherein the RNA linker further comprises a type II endonuclease recognition sequence.

6. The method of claim 1, wherein the method further comprises the step of generating a ditag by linking the end of a gene tag cleaved by the type IIs endonuclease to the end of another gene tag cleaved by the type IIs endonuclease.

7. The method of claim 6, wherein the method further comprises the step of amplifying the ditag using a primer that anneals to the RNA linker.

8. The method of claim 1, wherein the method further comprises the step of ligating an adapter having an arbitrary nucleotide sequence to the end of a gene tag cleaved by the type IIs endonuclease and amplifying the gene tag using primers that anneal to the RNA linker and the adapter.

9. A method for producing a concatemer of gene tags, wherein the method comprises the step of linking multiple gene tags generated by the method of claim 1.

10. A method for producing a concatemer of gene tags, wherein the method comprises the step of linking multiple ditags generated by the method of claim 6.

11. A method for determining the nucleotide sequence of a gene tag, wherein the method comprises the step of determining the nucleotide sequence of a concatemer produced by the method of claim 9 or 10.

12. A reagent kit for producing a gene tag, wherein the kit comprises:

(a) an RNA linker that comprises an oligonucleotide comprising a type IIs endonuclease recognition sequence;
(b) a reagent for linking the RNA linker with the CAP site of an RNA;
(c) a primer for cDNA second strand synthesis, which comprises an oligonucleotide that anneals to a cDNA synthesized using the RNA linker as a template; and
(d) a primer for cDNA first strand synthesis.

13. The kit of claim 12, wherein the primer for cDNA first strand synthesis is selected from the group consisting of:

(i) a random primer;
(ii) an oligo dT primer; and
(iii) a primer comprising a nucleotide sequence complementary to a particular mRNA.

14. A method for obtaining an expression profile of a gene in eukaryotic cells, wherein the method comprises the steps of:

(1) producing a gene tag by the method of claim 1;
(2) determining the nucleotide sequence of the gene tag of (1); and
(3) obtaining the expression profile by relating the determined nucleotide sequence to its frequency of occurrence.

**15.** A database of gene expression profiles constructed by accumulating information of gene expression profiles obtained by the method of claim 14.

**16.** A method for analyzing gene expression profiles, wherein the method comprises the step of obtaining gene expression profiles from different types of cells by the method of claim 14, comparing the gene expression profiles and selecting a gene tag whose frequency of occurrence differs among the cells.

**17.** A method for determining the transcriptional start site of a gene, wherein the method comprises the steps of:

(1) producing a gene tag by the method of claim 1;
(2) determining the nucleotide sequence of the gene tag of (1); and
(3) mapping the determined nucleotide sequence onto a genomic nucleotide sequence and identifying a region where the nucleotide sequences match as the transcriptional start site of the gene.

**18.** The method of claim 17, wherein the primer for cDNA first strand synthesis comprises a nucleotide sequence selected from the nucleotide sequence of a particular gene, wherein the method comprises determining the transcriptional start site of the gene.

**19.** A primer set for cDNA synthesis, wherein the primer set comprises a 3' primer that anneals to an arbitrary portion of a cDNA and a 5' primer for synthesizing a cDNA comprising a nucleotide sequence, or the complementary sequence thereto, determined by the steps of:

(1) producing a gene tag by the method of claim 1; and
(2) determining the nucleotide sequence of the gene tag of (1).

**20.** The primer set of claim 19, wherein the 3' primer is selected from the group consisting of:

(i) an oligo dT primer;
(ii) sequence information on a cDNA fragment; and
(iii) a primer comprising the nucleotide sequence of a gene tag adjacent to the type II endonuclease recognition sequence in the cDNA or the complementary sequence thereto.

**21.** A method for synthesizing a full-length cDNA, wherein the method comprises the steps of:

(a) carrying out complementary strand synthesis using an RNA or cDNA as a template and using a 3' primer comprising an oligo dT primer and a 5'primer for synthesizing a cDNA comprising a nucleotide sequence, or the complementary sequence thereto, determined by the steps of:

(1) producing a gene tag by the method of claim 1; and
(2) determining the nucleotide sequence of the gene tag of (1); and

(b) recovering a synthesized DNA as the full-length cDNA.

**22.** A full-length cDNA obtained by the method of claim 21.

**23.** A polypeptide comprising an amino acid sequence encoded by the full-length cDNA of claim 22.

**24.** An antibody which recognizes the polypeptide of claim 23.

**25.** A vector carrying and capable of expressing the coding region of the full-length cDNA of claim 22.

**26.** A transformant comprising and capable of expressing the vector of claim 25.

**27.** A method for producing the polypeptide of claim 23, wherein the method comprises the step of culturing the transformant of claim 26 and collecting an expressed product.

**28.** A method for producing the polypeptide of claim 23, wherein the method comprises the steps of:

(i) contacting an element supporting *in vitro* translation with a DNA construct comprising the coding region of the full-length cDNA of claim 22 operatively linked to a promoter; and
(ii) collecting an expressed product.

**29.** A method for synthesizing a cDNA comprising a nucleotide sequence from the 5' end of an mRNA, wherein the method comprises the steps of:

(a) carrying out complementary strand synthesis using an RNA or cDNA as a template and using a 3' primer comprising a nucleotide sequence complementary to an arbitrary region of an mRNA of interest and a 5' primer for synthesizing a cDNA comprising a nucleotide sequence, or the complementary strand thereto, determined by the steps of:

(1) producing a gene tag by the method of claim 1; and
(2) determining the nucleotide sequence of the gene tag of(1); and

(b) recovering a synthesized DNA as the cDNA comprising a nucleotide sequence from the 5' end of an mRNA.

**30.** A method for determining the 5' nucleotide sequence of an mRNA, wherein the method comprises the step of determining the nucleotide sequence of the cDNA recovered by the method of claim 29.

FIG. 1

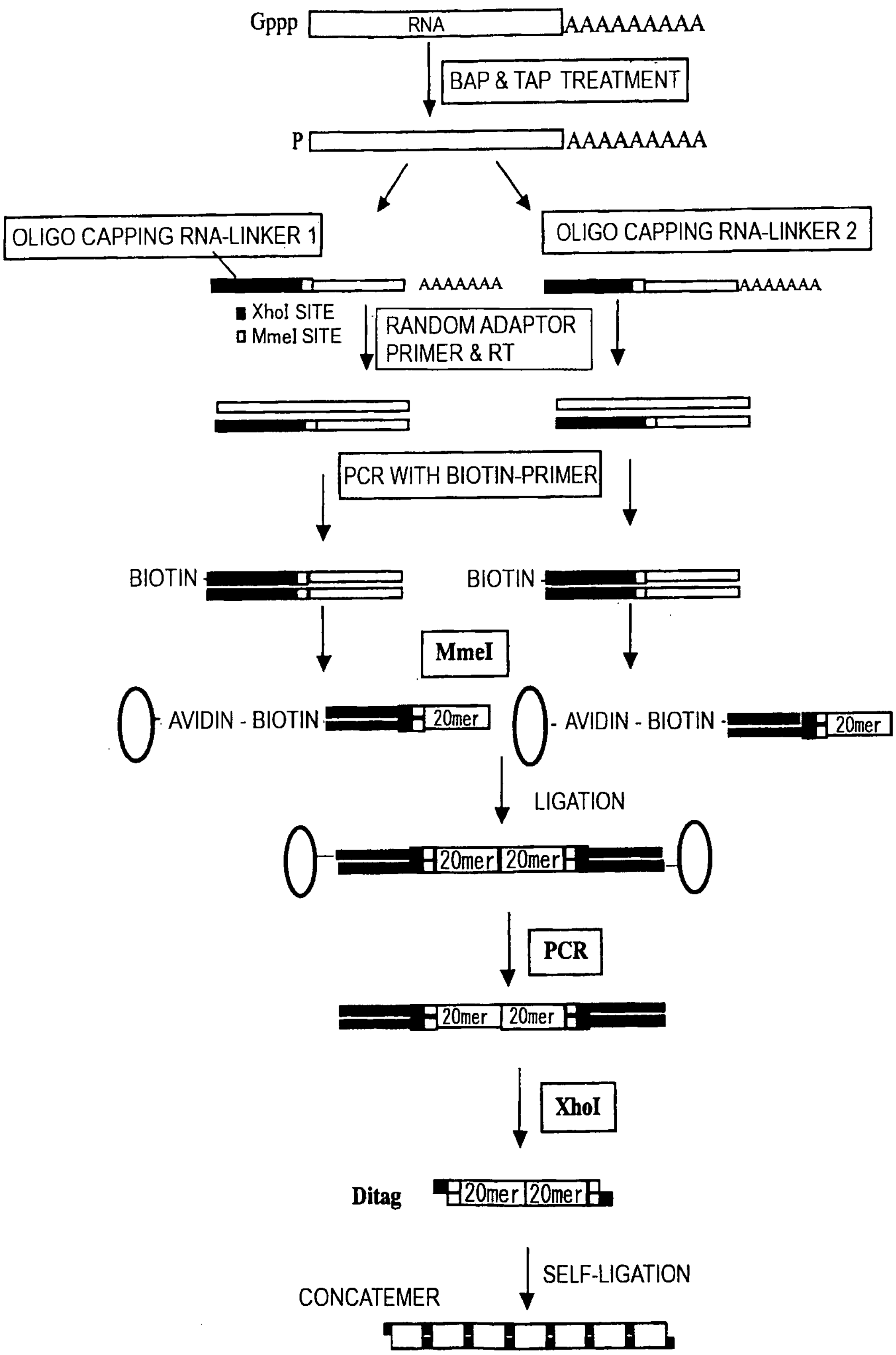
Gppp
RNA
AAAAAAAAA
BAP & TAP TREATMENT
P
AAAAAAAAA
OLIGO CAPPING RNA-LINKER 1
OLIGO CAPPING RNA-LINKER 2
AAAAAAA
AAAAAAA
XhoI SITE
MmeI SITE
RANDOM ADAPTOR PRIMER & RT
PCR WITH BIOTIN-PRIMER
BIOTIN
BIOTIN
MmeI
AVIDIN - BIOTIN
20mer
AVIDIN - BIOTIN
20mer
LIGATION
20mer
20mer
PCR
20mer
20mer
XhoI
Ditag
20mer
20mer
SELF-LIGATION
CONCATEMER

FIG. 2

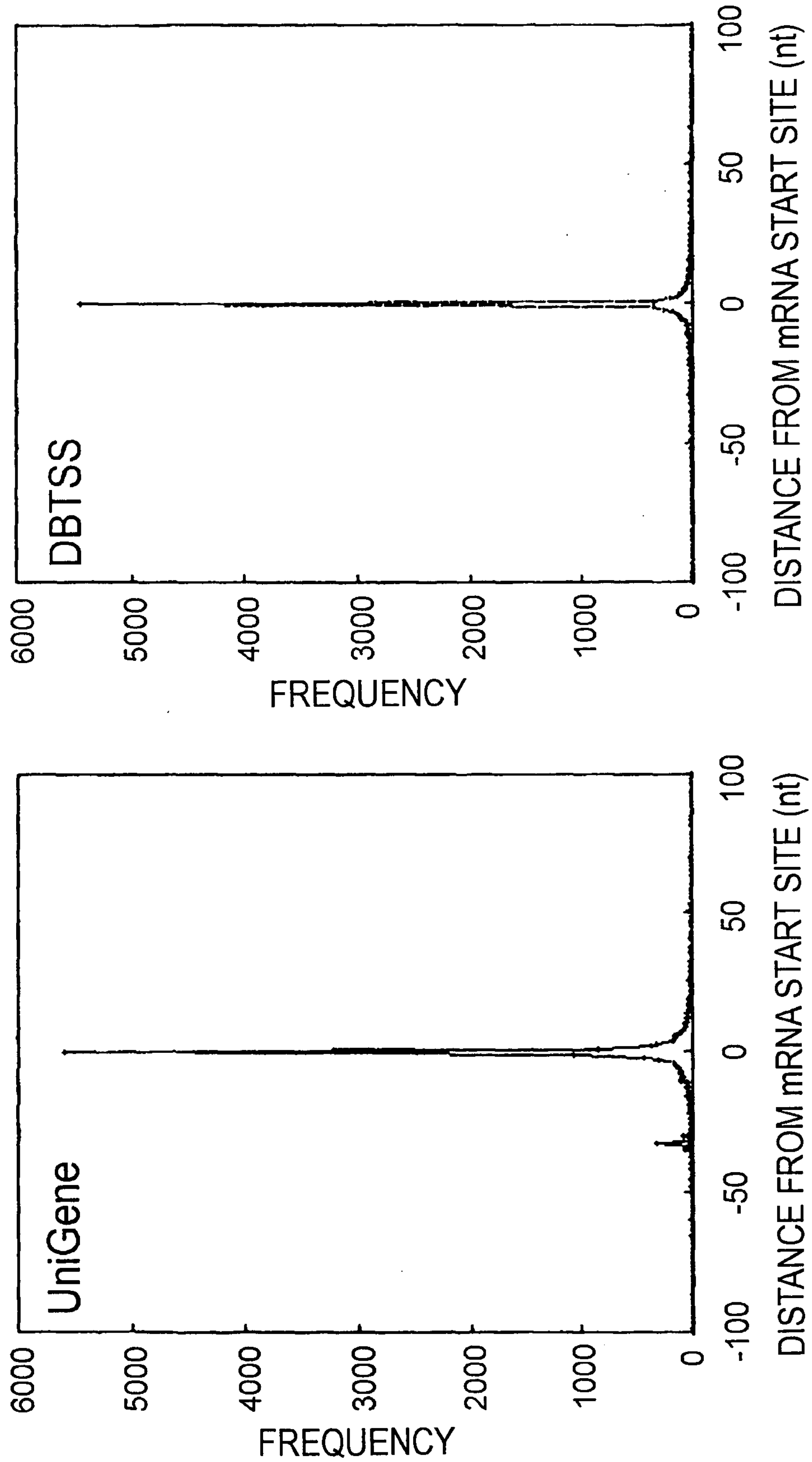
DBTSS
FREQUENCY
6000
5000
4000
3000
2000
1000
0
DISTANCE FROM mRNA START SITE (nt)
-100
-50
0
50
100
UniGene
FREQUENCY
6000
5000
4000
3000
2000
1000
0
DISTANCE FROM mRNA START SITE (nt)
-100
-50
0
50
100

FIG. 3

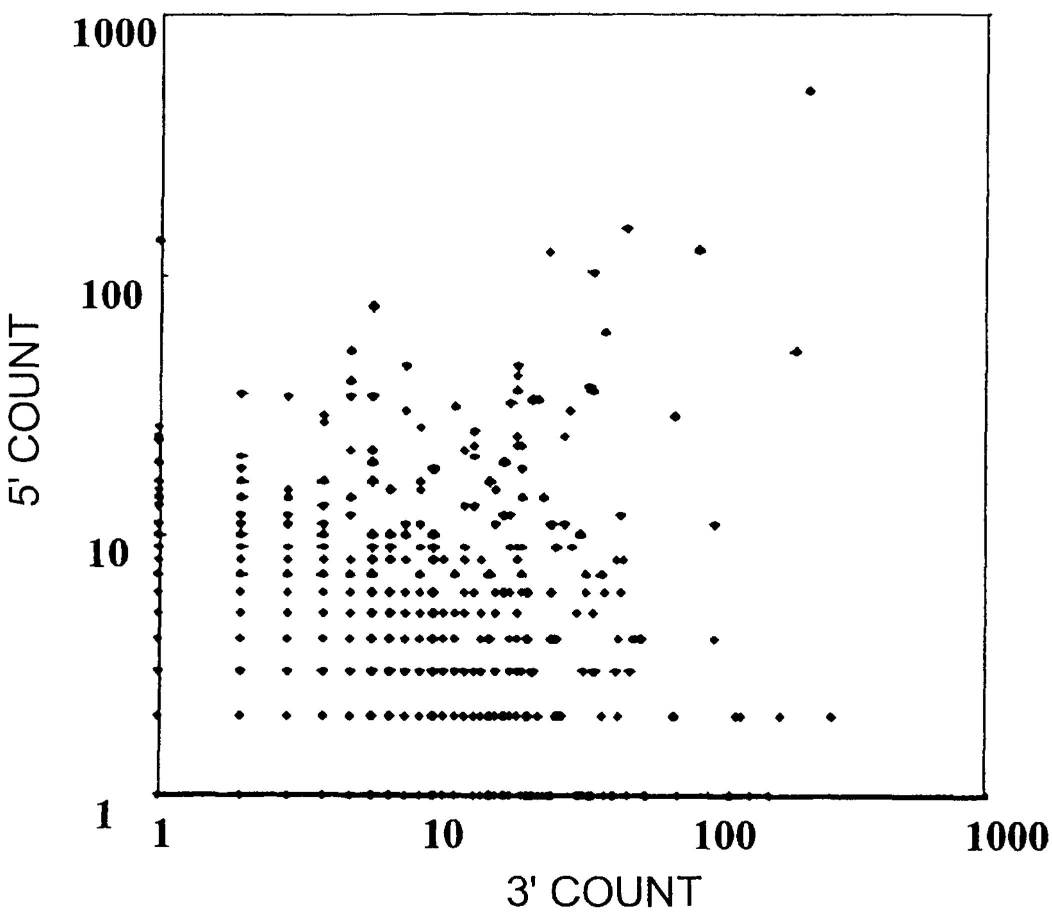

**INTERNATIONAL SEARCH REPORT**

International application No. PCT/JP2004/008174

A. CLASSIFICATION OF SUBJECT MATTER
Int.Cl$^7$ C12N15/09, C12N15/12, C12Q1/68, C07K14/47, C07K16/18, C12P21/02, C12N1/15, C12N1/19, C12N1/21, C12N5/10

According to International Patent Classification (IPC) or to both national classification and IPC

B. FIELDS SEARCHED

Minimum documentation searched (classification system followed by classification symbols)
Int.Cl$^7$ C12N15/09, C12N15/12, C12Q1/68, C07K14/47, C07K16/18, C12P21/02, C12N1/15, C12N1/19, C12N1/21, C12N5/10

Documentation searched other than minimum documentation to the extent that such documents are included in the fields searched

Electronic data base consulted during the international search (name of data base and, where practicable, search terms used)
BIOSIS/WPI(DIALOG), MEDLINE(STN), JSTPlus/JST7580(JOIS)

C. DOCUMENTS CONSIDERED TO BE RELEVANT

| Category* | Citation of document, with indication, where appropriate, of the relevant passages | Relevant to claim No. |
|---|---|---|
| X/<br>A | Gnatenko D.V. et al., "Transcript profiling of human platelets using microarray and serial analysis of gene expression", Blood, 15 March, 2003 (15.03.03), Vol.101, No.6, pages 2285 to 2293 | 19-30/<br>1-14,16-18 |
| X | Myers M.W. et al., "The human mid-size neurofilament subunit: a repeated protein sequence and the relationship of its gene to the intermediate filament gene family", EMBO J., 1987, Vol.6, No.6, pages 1617 to 1626 | 19-30 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 June, 2004 (25.06.04) | 13 July, 2004 (13.07.04) |
| Name and mailing address of the ISA/<br>Japanese Patent Office | Authorized officer |
| Facsimile No. | Telephone No. |

Form PCT/ISA/210 (second sheet) (January 2004)

INTERNATIONAL SEARCH REPORT

International application No. PCT/JP2004/008174

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-511002 A (UNIV JOHNS HOPKINS SCHOOL MEDICINE), 27 October, 1998 (27.10.98), & EP 761822 A2 & GB 2305241 A & WO 97/10363 A1 & AU 96655614 A & CA 2185379 A & AU 9670188 A & US 5695937 A & IE 80465 B & US 5866330 A & JP 2001-145495 A & JP 2001-155035 A & US 2003/0049653 A1 | 1-14,16-30 |
| A | Velculescu V.E. et al., "Serial analysis of gene expression", Science, 1995, Vol.270, No.5235, pages 484 to 487 | 1-14,16-30 |
| A | Maruyama K. et al., "Oligo-cappin: a simple method to replace the cap structure of eukaryotic mRNAs with oligoribonucleotides, Gene, 1994, Vol.138, Nos.1 to 2, pages 171 to 174 | 1-14,16-30 |
| A | Tucholski J. et al., "MmeI, a class-IIS restriction endonuclease: purification and characterization, Gene, 1995, Vol.157, Nos.1 to 2, pages 87 to 92 | 1-14,16-30 |

Form PCT/ISA/210 (continuation of second sheet) (January 2004)

**INTERNATIONAL SEARCH REPORT**

International application No.
PCT/JP2004/008174

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 15
because they relate to subject matter not required to be searched by this Authority, namely:
It is recognized that the invention as set forth in the above claim pertains to mere presentation of information.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

SEQUENCE LISTING

<110> POST GENOME INSTITUTE CO., LTD.

<120> METHOD OF OBTAINING GENE TAG

<130> PGI-A0301Y1P

<150> JP 2003-402306

<151> 2003-12-01

<150> JP 2004-6630

<151> 2004-01-14

<160> 69

<170> PatentIn version 3.1

<210> 1

<211> 48

<212> RNA

<213> Artificial

<220>

<223> an artificially synthesized RNA linker sequence

<400> 1

uuuggauuug cuggugcagu acaacuaggc uuaauacucg aguccgac 48

<210> 2

<211> 46

<212> RNA

<213> Artificial

<220>

<223> an artificially synthesized RNA linker sequence

<400> 2

uuucugcucg aauucaagcu ucuaacgaug uacgcucgag uccgac 46

<210> 3

<211> 42

<212> DNA

<213> Artificial

<220>

<223> an artificially synthesized primer sequence

<400> 3

gcggctgaag acggcctatg tggccttttt tttttttttt tt 42

<210> 4

<211> 32

<212> DNA

<213> Artificial

<220>

<223> an artificially synthesized primer sequence

<220>

<221> misc_feature

<222> (26)..(31)

<223> "n"=a, t, g or c

<400> 4

gcggctgaag acggcctatg tggccnnnnn nc 32

<210> 5

<211> 33

<212> DNA

<213> Artificial

<220>

<223> an artificially synthesized primer sequence

<220>

<221> misc_feature

<222> (1)..(1)

<223> Label biotin

<400> 5

ggatttgctg gtgcagtaca actaggctta ata 33

<210> 6

<211> 31

<212> DNA

<213> Artificial

<220>

<223> an artificially synthesized primer sequence

<220>

<221> misc_feature

<222> (1)..(1)

<223> Label biotin

<400> 6

ctgctcgaat tcaagcttct aacgatgtac g 31

<210> 7

<211> 21

<212> DNA

<213> Artificial

<220>

<223> an artificially synthesized primer sequence

<400> 7

gcggctgaag acggcctatg t 21

<210> 8

<211> 27

<212> DNA

<213> Artificial

<220>

<223> an artificially synthesized primer sequence

<400> 8

ggatttgctg gtgcagtaca actaggc 27

<210> 9

<211> 27

<212> DNA

<213> Artificial

<220>

<223> an artificially synthesized primer sequence

<400> 9

ctgctcgaat tcaagcttct aacgatg 27

<210> 10

<211> 18

<212> DNA

<213> Homo sapiens

<400> 10

acatctgacc tcatggag 18

<210> 11

<211> 18

<212> DNA

<213> Homo sapiens

<400> 11

ctctttcctt gcctaacg 18

<210> 12
<211> 18
<212> DNA
<213> Homo sapiens

<400> 12
tacctggttg atcctgcc 18

<210> 13
<211> 18
<212> DNA
<213> Homo sapiens

<400> 13
cttttcctgt ggcagcag 18

<210> 14
<211> 18
<212> DNA
<213> Homo sapiens

<400> 14
ctcttccgcc gtcgtcgc 18

<210> 15
<211> 18
<212> DNA
<213> Homo sapiens

<400> 15
ctcattgaac tcgcctgc 18

<210> 16
<211> 18
<212> DNA
<213> Homo sapiens

<400> 16
ctggttgatc ctgccagt 18

<210> 17
<211> 18
<212> DNA
<213> Homo sapiens

<400> 17
ctcagtcgcc gctgccag 18

<210> 18
<211> 18
<212> DNA
<213> Homo sapiens

<400> 18
ctttcactgc aaggcggc 18

<210> 19
<211> 18
<212> DNA
<213> Homo sapiens

<400> 19
acgctgtgac agccacac 18

<210> 20
<211> 18
<212> DNA

<213> Homo sapiens

<400> 20
gtgacagcca cacgcccc 18

<210> 21
<211> 18
<212> DNA
<213> Homo sapiens

<400> 21
aacggctagc ctgaggag 18

<210> 22
<211> 18
<212> DNA
<213> Homo sapiens

<400> 22
agtagcagca gcgccggg 18

<210> 23
<211> 18

<212> DNA

<213> Homo sapiens

<400> 23

attcctagtt aaggcggc 18

<210> 24

<211> 18

<212> DNA

<213> Homo sapiens

<400> 24

aattgtgttc gcagccgc 18

<210> 25

<211> 18

<212> DNA

<213> Homo sapiens

<400> 25

atatttctta ctctctcg 18

<210> 26

<211> 18

<212> DNA

<213> Homo sapiens

<400> 26

ctcagtcgcc gctgccaa 18

<210> 27

<211> 18

<212> DNA

<213> Homo sapiens

<400> 27

aaaacggcca gcctgagg 18

<210> 28

<211> 18

<212> DNA

<213> Homo sapiens

<400> 28

ctctctttca ctgcaagg 18

<210> 29

<211> 18

<212> DNA

<213> Homo sapiens

<400> 29

aatttctacg cgcaccgg 18

<210> 30

<211> 18

<212> DNA

<213> Homo sapiens

<400> 30

accgccgaga ccgcgtcc 18

<210> 31

<211> 18

<212> DNA

<213> Homo sapiens

<400> 31

agacgcagag tagattgt 18

<210> 32

<211> 18

<212> DNA

<213> Homo sapiens

<400> 32

agttcgatcg gtagcggg 18

<210> 33

<211> 18

<212> DNA

<213> Homo sapiens

<400> 33

agttctcggg cgtacggc 18

<210> 34

<211> 18

<212> DNA

<213> Homo sapiens

<400> 34

agttgcttca gcgtcccg 18

<210> 35

<211> 18

<212> DNA

<213> Homo sapiens

<400> 35

attaaacggt tgcaggcg 18

<210> 36

<211> 18

<212> DNA

<213> Homo sapiens

<400> 36

ccggccgggg ggcgggcg 18

<210> 37

<211> 18

<212> DNA

<213> Homo sapiens

<400> 37

cctttggct ctctgacc 18

<210> 38

<211> 18

<212> DNA

<213> Homo sapiens

<400> 38

ctcagtacag ctccggcc 18

<210> 39

<211> 18

<212> DNA

<213> Homo sapiens

<400> 39

ctctttcggc cgcgctgg 18

<210> 40

<211> 45

<212> DNA

<213> Artificial

<220>

<223> an artificially synthesized DNA linker sequence

<400> 40

tttggatttg ctggtgcagt acaactaggc ttaatatccg acatg 45

<210> 41

<211> 38

<212> DNA

<213> Artificial

<220>

<223> an artificially synthesized DNA linker sequence

<220>

<221> misc_feature

<222> (38)..(38)

<223> C7-amino-modified

<400> 41

tcggatatta agcctagttg tactgcacca gcaaatcc 38

<210> 42

<211> 43

<212> DNA

<213> Artificial

<220>

<223> an artificially synthesized DNA linker sequence

<400> 42

tttctgctcg aattcaagct tctaacgatg tacgtccgac atg 43

<210> 43

<211> 36

<212> DNA

<213> Artificial

<220>

<223> an artificially synthesized DNA linker sequence

<220>

<221> misc_feature

<222> (36)..(36)

<223> C7-amino-modified

<400> 43

tcggacgtac atcgttagaa gcttgaattc gagcag 36

<210> 44

<211> 18

<212> DNA

<213> Homo sapiens

<400> 44

gctgtgacag ccacacgc 18

<210> 45

<211> 18

<212> DNA

<213> Homo sapiens

<400> 45

ctttttcgca acgggttt 18

<210> 46

<211> 18

<212> DNA

<213> Homo sapiens

<400> 46

cctcctcatc acacgccg 18

<210> 47

<211> 18

<212> DNA

<213> Homo sapiens

<400> 47

ctttctgccc gtggacgc 18

<210> 48

<211> 18

<212> DNA

<213> Homo sapiens

<400> 48

agtacagctc cggccgcc 18

<210> 49

<211> 18

<212> DNA

<213> Homo sapiens

<400> 49

cacctgtttg caggctgc 18

<210> 50

<211> 18

<212> DNA

<213> Homo sapiens

<400> 50

gtccgtactg cagagccg 18

<210> 51

<211> 18

<212> DNA

<213> Homo sapiens

<400> 51

atttcgtctt agccacgc 18

<210> 52

<211> 18

<212> DNA

<213> Homo sapiens

<400> 52
aggcattgag gcagccag 18

<210> 53
<211> 18
<212> DNA
<213> Homo sapiens

<400> 53
agtgggcgga ccgcgcgg 18

<210> 54
<211> 18
<212> DNA
<213> Homo sapiens

<400> 54
cccaatttct acgcgcac 18

<210> 55
<211> 18
<212> DNA
<213> Homo sapiens

<400> 55
ctcgttgcgc agtagtgc 18

<210> 56
<211> 18
<212> DNA
<213> Homo sapiens

<400> 56
gtgctgcagc cgctgccg 18

<210> 57
<211> 18
<212> DNA
<213> Homo sapiens

<400> 57
catttcgtct tagccacg 18

<210> 58
<211> 18
<212> DNA

<213> Homo sapiens

<400> 58

ctctttccct aagcagcc 18

<210> 59

<211> 18

<212> DNA

<213> Homo sapiens

<400> 59

gactaatttg ttggcggc 18

<210> 60

<211> 18

<212> DNA

<213> Homo sapiens

<400> 60

acctcattca tttctacc 18

<210> 61

<211> 18

<212> DNA

<213> Homo sapiens

<400> 61

cctttctgcc cgtggacg 18

<210> 62

<211> 18

<212> DNA

<213> Homo sapiens

<400> 62

agtatctgtg ggtacccg 18

<210> 63

<211> 18

<212> DNA

<213> Homo sapiens

<400> 63

caatttctac gcgcaccg 18

<210> 64

<211> 18

<212> DNA

<213> Homo sapiens

<400> 64

gcacacagcc atccatcc 18

<210> 65

<211> 18

<212> DNA

<213> Homo sapiens

<400> 65

agtgacgcgt attgcctg 18

<210> 66

<211> 18

<212> DNA

<213> Homo sapiens

<400> 66

ctctttccag ccagcgcc 18

<210> 67

<211> 18

<212> DNA

<213> Homo sapiens

<400> 67

cttttccgcc cgctcccc 18

<210> 68

<211> 18

<212> DNA

<213> Homo sapiens

<400> 68

gcgtcttgtt cttgcctg 18

<210> 69

<211> 18

<212> DNA

<213> Homo sapiens

<400> 69

atatagaggc tgggggtg 18